(19) Europäisches Patentamt
European Patent Office
Office européen des brevets

(11) EP 2 429 643 B1

(12) EUROPEAN PATENT SPECIFICATION

(45) Date of publication and mention
of the grant of the patent:
23.08.2017 Bulletin 2017/34

(51) Int Cl.:
A61N 1/36 (2006.01)
G06N 99/00 (2010.01)
G06K 9/62 (2006.01)
G06F 19/00 (2011.01)

(21) Application number: 10702191.7

(22) Date of filing: 26.01.2010

(86) International application number:
PCT/US2010/022114

(87) International publication number:
WO 2010/126624 (04.11.2010 Gazette 2010/44)

(54) **PATIENT STATE DETECTION BASED ON SUPPORT VECTOR MACHINE BASED ALGORITHM**

ERKENNUNG EINES PATIENTENZUSTANDS NACH EINEM ALGORITHMUS AUF DER BASIS EINER SUPPORT-VECTOR-MASCHINE

DÉTECTION D'ÉTAT DE PATIENT BASÉE SUR UN ALGORITHME BASÉ SUR UNE MACHINE VECTEUR DE SOUTIEN

(84) Designated Contracting States:
AT BE BG CH CY CZ DE DK EE ES FI FR GB GR HR HU IE IS IT LI LT LU LV MC MK MT NL NO PL PT RO SE SI SK SM TR

(30) Priority: 30.04.2009 US 174355 P

(43) Date of publication of application:
21.03.2012 Bulletin 2012/12

(73) Proprietor: Medtronic, Inc.
Minneapolis MN 55432-5604 (US)

(72) Inventors:
• CARLSON, David, L.
Fridley
MN 55421 (US)
• DENISON, Timothy, J.
Minneapolis
MN 55410 (US)
• SHOEB, Ali, H.
Winchester
MA 01890 (US)

(74) Representative: Hughes, Andrea Michelle
Dehns
St Bride's House
10 Salisbury Square
London EC4Y 8JD (GB)

(56) References cited:
WO-A1-2004/100781    WO-A1-2005/117693
WO-A1-2007/054841    WO-A2-02/096282
WO-A2-2006/017153    US-B1- 6 658 287

• MADEVSKA-BOGDANOVA A ET AL: "A new approach of modifying SVM outputs", NEURAL NETWORKS, 2000. IJCNN 2000, PROCEEDINGS OF THE IEEE-INNS-ENNS I NTERNATIONAL JOINT CONFERENCE ON 24-27 JULY 2000, PISCATAWAY, NJ, USA,IEEE, vol. 6, 24 July 2000 (2000-07-24), pages 395-398, XP010505019, ISBN: 978-0-7695-0619-7
• GONZALEZ-VELLON B ET AL: "Support vector machines for seizure detection", SIGNAL PROCESSING AND INFORMATION TECHNOLOGY, 2003. ISSPIT 2003. PROCE EDINGS OF THE 3RD IEEE INTERNATIONAL SYMPOSIUM ON DARMSTADT, GERMANY 14-17 DEC. 2003, PISCATAWAY, NJ, USA,IEEE, 14 December 2003 (2003-12-14), pages 126-129, XP010729110, ISBN: 978-0-7803-8292-3

## Description

### TECHNICAL FIELD

The disclosure relates to medical devices, and, more particularly, patient state detection by medical devices.

### BACKGROUND

Implantable medical devices, such as electrical stimulators or therapeutic agent delivery devices, may be used in different therapeutic applications, such as deep brain stimulation (DBS), spinal cord stimulation (SCS), pelvic stimulation, gastric stimulation, peripheral nerve stimulation, functional electrical stimulation or delivery of pharmaceutical agent, insulin, pain relieving agent or antiinflammatory agent to a target tissue site within a patient. A medical device may be used to deliver therapy to a patient to treat a variety of symptoms or patient conditions such as chronic pain, tremor, Parkinson's disease, other types of movement disorders, seizure disorders (e.g., epilepsy), urinary or fecal incontinence, sexual dysfunction, obesity, psychiatric disorders, gastroparesis or diabetes. In some therapy systems, an implantable electrical stimulator delivers electrical therapy to a target tissue site within a patient with the aid of one or more electrodes, which may be deployed by medical leads. In addition to or instead of electrical stimulation therapy, a medical device may deliver a therapeutic agent to a target tissue site within a patient with the aid of one or more fluid delivery elements, such as a catheter or a therapeutic agent eluting patch.

An external or implantable medical device may be configured to sense one or more patient parameters, such as a physiological signal, patient activity level or patient posture. In some examples, detection of a patient state based on the one or more sensed physiological parameters may be used to control therapy delivery. WO 2006/017153, WO 2004/100781, WO 02/096282, WO 2005/117693 and WO 2007/054841 are all concerned with determining a state of a patient.

### SUMMARY

In general, the disclosure is directed to patient state detection with a classification algorithm that is determined based on supervised machine learning. The supervised machine learning can be applied, for example, using a support vector machine as in the current invention, which is defined in claim 1, or another artificial neural network techniques. Supervised machine learning is implemented to generate a classification boundary during a learning phase based on values of two or more features of one or more patient parameter signals known to be indicative of the patient being in the patient state and feature values of one or more patient parameter signals known to be indicative of the patient not being in the patient state. A feature is a characteristic of the patient parameter signal, such as an amplitude or an energy level in a specific frequency band. The classification boundary delineates the feature values indicative of the patient being in the patient state and feature values indicative of the patient not being in the patient state.

Once the classification boundary is determined based on the known patient state data, a medical device may use the boundary to detect when the patient is in a particular patient state by determining the side of the boundary on which a particular feature value extracted from a sensed patient parameter signal lies. The patient state detection may be used to control various courses of action, such as controlling therapy delivery, generating a patient notification or evaluating a patient condition. In addition, various metrics for monitoring and evaluating a patient condition can be determined based on the classification boundary and a signal indicative of a patient parameter.

The invention is directed to a system comprising:means configured to generate a signal indicative of a parameter of a patient, means configured to determine a feature vector based on the signal, means configured to apply a support vector machine based algorithm to classify a patient state based on the feature vector, wherein the support vector machine based algorithm defines a classification boundary, means configured to determine a distance between the feature vector and the classification boundary after classifying the patient state based on the feature vector and means configured to determine an evaluation metric for the patient state based on the distance, and wherein the evaluation metric indicates a severity of the patient state.

The details of one or more examples of the disclosure are set forth in the accompanying drawings and the description below.

### BRIEF DESCRIPTION OF DRAWINGS

FIG. 1 is a conceptual diagram illustrating an example deep brain stimulation (DBS) system.
FIG. 2 is functional block diagram illustrating components of an example medical device.

FIG. 3 is a functional block diagram illustrating components of an example medical device programmer.

FIG. 4 is a flow diagram of an example technique for training a support vector machine (SVM) algorithm to respond to future patient parameter signal inputs and classify the patient parameter signal inputs as being representative of a first patient state or a second patient state.

FIG. 5 is a conceptual illustration of the functionality of a computing device that implements an SVM-based algorithm for determining a classification boundary for classifying a sensed patient parameter signal as indicative of a first patient state or a second patient state.

FIG. 6 illustrates an example of a feature space that includes a linear classification boundary.

FIG. 7 illustrates an example of a feature space that includes two linear classification boundaries.

FIGS. 8A and 8B illustrate examples of nonlinear classification boundaries.

FIG. 9 is a flow diagram illustrating an example technique for determining a patient state based on a real-time or stored patient parameter signal.

FIG. 10 is a conceptual illustration of the technique with which a medical device determines a patient state based on a signal indicative of a patient parameter.

FIG. 11 is a flow diagram illustrating an example technique for monitoring a patient state based on a trajectory of feature vectors within a feature space.

FIG. 12 is a flow diagram of an example technique a processor may implement to determine which of three patient states a sensed physiological signal indicates.

FIG. 13 is a flow diagram illustrating an example technique a processor may implement to determine an evaluation metric with the aid of a classification boundary generated using a SVM algorithm.

FIGS. 14A and 14B are conceptual illustrations of a feature space, illustrating how a distance between a classification boundary and a determined feature vector may be determined.

FIG. 15 is an example of a data structure that associates a plurality of distances of a feature vector from a classification boundary to a respective severity metric.

FIGS. 16 and 17 are conceptual block diagrams of example circuitry of a sensing module of a medical device.

FIG. 18 is a table that compares different sensing capabilities based on the seizure detection latency, sensitivity, and the number of false detections per day.

FIG. 19 is a table that compares a current drain for seizure detection algorithms that were implemented using a prototype implantable device.

## DETAILED DESCRIPTION

Detecting one or more patient states may be useful for various purposes, such as monitoring and/or evaluating a patient condition, controlling therapy delivery to a patient, generating a patient or other user notification, data logging, initiating recording of a patient parameter, and the like. Techniques described herein include detecting a patient state based on one or more sensed patient parameters (also referred to as patient state biomarkers) with a classification algorithm that is determined based on any one or more machine learning techniques implemented by a computing device (e.g., a medical device programmer, a medical device or another computing device configured to receive patient parameter signals and generate a classification algorithm based on the signals). Example machine learning techniques include, but are not limited to, a genetic algorithm, an artificial neural network (e.g., based on a support vector machine (SVM), Bayesian classifiers, and the like) or other supervised machine learning techniques. Therefore, the patient state detection algorithm may be referred to as a supervised machine learning-based algorithm in the sense that a classification boundary that is used to classify patient parameters as indicative of a patient state is generated using supervised machine learning.

The computing device implementing (or applying) the supervised machine learning algorithm receives a signal indicative of a patient parameter (e.g., a physiological parameter or a patient posture or activity level) and extracts signal characteristics directly from the signals or from a parameterized signal or data generated based on the raw patient parameter signal in order to generate the classification algorithm. The signal characteristics are processed via the supervised machine learning algorithm in order to generate the classification boundary.

The description of some examples of devices, systems, and techniques described herein refer to patient state detection using a classification boundary determined based on a SVM, which can be referred to as a SVM-based algorithm. In other examples, the devices, systems, and techniques described herein can utilize other types of patient state classification algorithms, such as classification algorithms that are determined (or generated) based on other supervised machine learning techniques. The supervised machine learning techniques generate a classification boundary based on training data (e.g., a patient parameter signal) from known occurrences of the patient state, where the classification boundary is used to predict or detect the occurrence of the patient state or evaluate the patient state, as described herein with respect to SVM-based algorithms.

In the techniques described herein, a patient state determination is made by determining the side of the classification boundary on which a feature vector extracted from a sensed patient parameter signal lies. A feature can be a patient parameter signal characteristic, and a feature vector includes two or more features. Thus, a feature vector determined based on a sensed patient parameter signal includes respective values for each of the features. Examples of signal characteristics include a morphology of the signal (e.g., amplitude, slope, frequency, peak value, trough value, or other traits of the signal) or the spectral characteristics of the signal (e.g., frequency band power level, a ratio of power levels, and the like). Each side of the classification boundary is associated with a different patient state. The classification boundary may separate feature vectors that are indicative of the patient state and feature vectors that are not indicative of the patient state. As described in further detail below, a classification boundary can be a linear boundary or a non-linear boundary. Moreover, the boundary can extend in a plurality of directions and traverse a multi-dimensional space (e.g., a two dimensional feature space, a three-dimensional feature space, a four dimensional feature space or more depending upon the number of features present in the feature vectors used to classify the patient state).

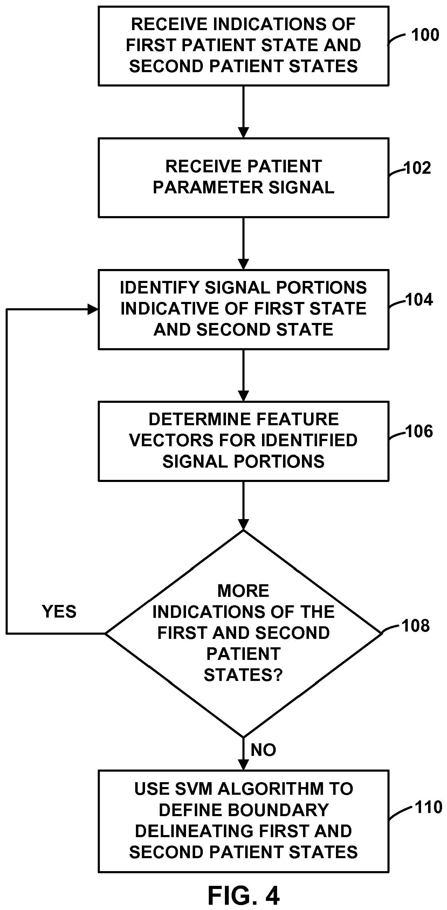

The techniques described herein also include determining the classification boundary with the aid of a SVM algorithm implemented by a computing device, such as a medical data computing device implemented in a general purpose computer, a medical device programmer, or an medical device, e.g., an implantable medical therapy or sensing device. As described below with reference to FIG. 4, the SVM algorithm uses features that are indicative of a known patient state to determine the classification boundary.

In some examples, the patient state includes a movement state and/or a non-movement state. A movement state may include a state in which a patient is intending on moving, is attempting to initiate movement or has initiated movement, and non-movement state may include a state in which the patient is not intending on moving, is not attempting to initiate movement. If the patient is afflicted with a movement disorder or other neurodegenerative impairment, the performance of certain motor tasks by the patient may be difficult. Accordingly, detecting whether a patient is in a movement state may be useful for controlling therapy delivery to a patient and providing movement disorder therapy to the patient in a closed-loop manner.

Therapy delivery, such as delivery of electrical stimulation therapy, a fluid delivery therapy (e.g., delivery of a pharmaceutical agent), fluid suspension delivery, or delivery of an external cue may improve the performance of motor tasks by the patient that may otherwise be difficult. These tasks may include at least one of initiating movement, maintaining movement, grasping and moving objects, improving gait associated with narrow turns, and so forth.

In other examples, the patient state includes a state in which one or more symptoms of a movement disorder are present. Symptoms of movement disorders include, for example, limited muscle control, motion impairment or other movement problems, such as rigidity, bradykinesia, rhythmic hyperkinesia, nonrhythmic hyperkinesia, and akinesia. In some cases, the movement disorder may be a symptom of Parkinson's disease. However, the movement disorder may be attributable to other patient conditions. By determining when the patient is experiencing symptoms of a movement disorder, a therapy system can provide on demand therapy to help manage the symptoms and improve patient movement as the therapy is needed or desired by the patient.

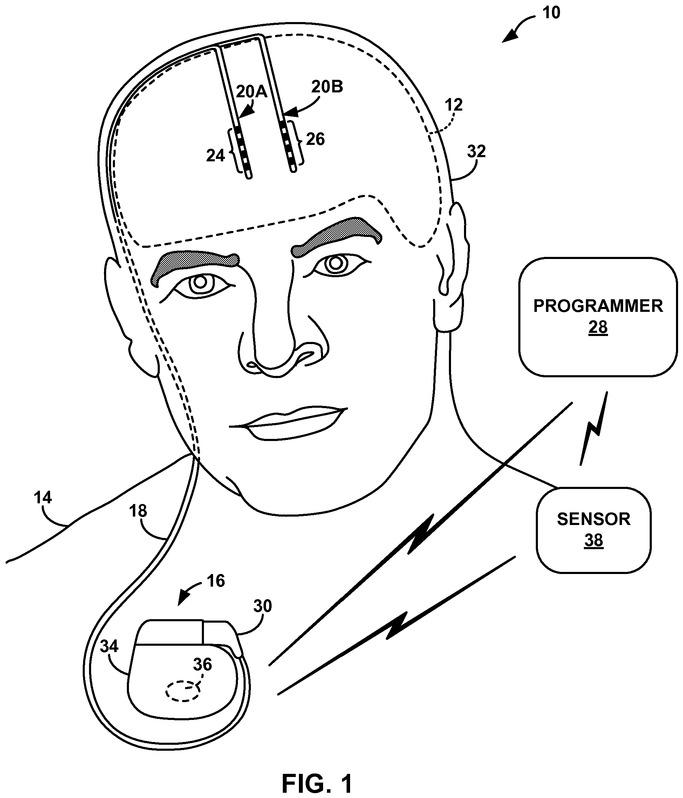

In examples in which the patient state includes a movement or non-movement state, the one or more signals indicative of a patient parameter that are used to determine the patient state may include, but are not limited to, bioelectrical brain signals, such as an electroencephalogram (EEG) signal, electrocorticogram (ECoG) signal, a local field potential (LFP) sensed from within one or more regions of a patient's brain and/or action potentials from single cells within the patient's brain. LFPs represent the ensemble activity of thousands to millions of cells in an *in vivo* neural population, and can be obtained via electrodes implanted within a brain of a patient (e.g., as shown in FIG. 1).

Low-frequency power fluctuations of discrete frequency bands in LFPs provide useful biomarkers for discriminating between brain states. Relevant biomarkers for differentiating between different patient states may span a relatively broad frequency spectrum, from about 1 Hertz (Hz) oscillations in a sleep state of a patient to greater than 500 Hz (e.g., "fast ripples" in the hippocampus) in other patient states. The biomarkers for various patient states may have widely varying bandwidths.

Other signals that may be used to determine a patient state in accordance with techniques described herein include signals generated by a motion sensor (e.g., a one-axis, two-axis or three-axis accelerometer, a gyroscope, a pressure transducer, or a piezoelectric crystal) or another type of sensor that generates a signal indicative of a patient parameter (e.g., physiological parameters such as blood pressure, tissue perfusion, heart rate, respiratory rate, muscle activity, electrodermal activity, body temperature, and the like).

A patient state may also include a mood state, which may be a symptom of a psychiatric disorder with which a patient is afflicted. For example, a patient mood state can be as an anxious state, a non-anxious mood state, a depressive state, a non-depressive mood state, a manic state, a non-manic state, a panic state, a non-panic state, and the like. Examples of psychiatric disorders that therapy system 10 may be useful for managing include major depressive disorder (MDD), bipolar disorder, anxiety disorders (e.g., post traumatic stress disorder, obsessive-compulsive disorder (OCD), panic disorder), or dysthymic disorder.

Detecting a mood state of a patient may be useful for, among other things, determining the severity or progression of a psychiatric disorder of a patient, formulating a therapy regimen for the patient, and controlling therapy delivery to the patient (e.g., activating therapy delivery, turning therapy off or adjusting one or more therapy delivery parameters). Detected patient mood states and, in some examples, patient parameters observed during the patient mood state can be stored by a device for later analysis by a clinician. Automatically determining patient mood states throughout an evaluation period may be more indicative of the status of the psychiatric disorder compared to relying on patient input indicative of the patient mood states.

In examples in which the patient state includes a patient mood state, the one or more signals indicative of a patient parameter that are used to determine the patient state may include, but are not limited to, bioelectrical brain signals. Instead of or in addition to the bioelectrical brain signals, the signals with which the patient mood state may be detected include, but are not limited to, signals indicative of a heart rate (e.g., as indicated by an electrocardiogram, electrogram, or a pulse oximeter), respiratory rate (e.g., as indicated by a transthoracic impedance sensor or a pulse oximeter), electrodermal activity (e.g., skin conductance level), changes in facial expression (e.g., as indicated by a facial electromyogram (EMG), facial flushing (e.g., as indicated by thermal sensing) or fatigue (e.g., as indicated by facial expression). As described in U.S. Patent Application Serial No. 12/426,065 by Giftakis et al., which is entitled "ANALYZING A WASHOUT PERIOD CHARACTERISTIC FOR PSYCHIATRIC DISORDER THERAPY DELIVERY" and was filed on April 17, 2009, these different physiological parameters can change as a function of a patient mood state, and, therefore, can be used to detect or determine a patient mood state.

A patient state may also include a posture state, which can refer to a state in which the patient is occupying particular patient posture or a combination of posture and activity. A posture state can include, for example, an upright posture state or a lying down posture state, where the upright posture state may be sub-categorized as upright and active or upright and inactive. Other posture states, such as lying down posture states, may or may not have an activity component. However, the lying down posture state can have other components. For example, the patient state may be a lying front posture state in which the patient is lying on a front side (e.g., a ventral side) of the body, a lying back posture state in which the patient is lying on a back side (e.g., a dorsal side) of the body, lying right posture state in which the patient is lying on a right side of the body, and a lying left posture state in which the patient is lying on a left side of the body.

Detection of a patient posture state may be useful for providing posture responsive therapy delivery to the patient. Changes in posture state may cause changes in efficacy of therapy delivery due to changes in distances between electrodes or other therapy delivery elements, e.g., due to temporary migration of leads or catheters caused by forces or stresses associated with different postures, or from changes in compression of patient tissue in different posture states. In addition, posture state changes may present changes in symptoms or symptom levels, e.g., pain level. To maintain therapeutic efficacy, it may be desirable to adjust one or more therapy parameter values based on different patient posture states, e.g., different posture s and/or activities engaged in by the patient.

A medical device may adjust therapy by modifying values for one or more therapy parameters, e.g., by specifying adjustments to a specific therapy parameter or by selecting different therapy programs or groups of programs that define different sets of therapy parameter values. That is, a therapy adjustment may be accomplished by selecting or adjusting parameter values for a current program (including parameters such as amplitude, pulse width, pulse rate, electrode combination, electrode polarity) or by selecting a different therapy program. In some examples, the medical device automatically makes the adjustments to one or more therapy parameter values based on a detected patient posture state.

In examples in which the patient state includes a patient posture state, the one or more signals indicative of a patient parameter may be generated by a motion sensor (e.g., a one-axis, two-axis or three-axis accelerometer, a gyroscope, a pressure transducer, or a piezoelectric crystal) that generates a signal indicative of the patient posture state. Instead of or in addition to the motion sensor, the signal may be indicative of an intracranial pressure, which may change as patient posture changes.

In some examples, a patient state includes a seizure state, in which one or more symptoms of a seizure of a patient are present, and a non-seizure state, in which the patient is not having a seizure. In some examples, the seizure state can also include a state in which a seizure is likely to occur. However, in other examples, the seizure state includes a state in which the patient is actually experiencing a seizure. This may be useful for, for example, evaluating a patient condition and generating a record of the patient's seizure activity.

Each of the patient states described herein may be detected alone or in combination with each other using the systems, devices, and techniques described herein. The examples described herein describe detecting a patient state based on a bioelectrical brain signal. In other examples, the techniques described herein are also applicable to detecting a patient state based on other types of signals indicative of a patient parameter, such as the other types of signals referenced above.

FIG. 1 is a conceptual diagram illustrating an example therapy system 10 that is implanted proximate to brain 12 of patient 14 in order to help manage a patient condition, such as pain, psychiatric disorder, movement disorder or seizure disorder. While patient 14 is generally referred to as a human patient, other mammalian or non-mammalian patients are also contemplated.

Therapy system 10 includes implantable medical device (IMD) 16, lead extension 18, leads 20A and 20B with respective sets of electrodes 24, 26, and medical device programmer 28. IMD 16 includes a therapy module that delivers electrical stimulation therapy to one or more regions of brain 12 via leads 20A and 20B (collectively referred to as "leads 20"). In the example shown in FIG. 1, therapy system 10 may be referred to a deep brain stimulation (DBS) system because IMD 16 provides electrical stimulation therapy directly tissue within brain 12, e.g., a tissue site under the dura mater of brain 12. In other examples, leads 20 may be positioned to deliver therapy to a surface of brain 12 (e.g., the cortical surface of brain 12). In addition, in some examples, DBS system 10 may include one lead or more than two leads.

In the example shown in FIG. 1, IMD 16 may be implanted within a subcutaneous pocket near a chest of patient 14. In other examples, IMD 16 may be implanted within other regions of patient 14, such as a subcutaneous pocket in the abdomen of patient 14 or proximate the cranium of patient 14. Implanted lead extension 18 is coupled to IMD 16 via connector block 30, which may include, for example, electrical contacts that electrically couple to respective electrical contacts on lead extension 18. The electrical contacts electrically couple the electrodes carried by leads 20 to IMD 16. Lead extension 18 traverses from the implant site of IMD 16 within a chest cavity of patient 14, along the neck of patient 14 and through cranium 32 of patient 14 to access brain 12.

Leads 20 may be positioned to deliver electrical stimulation to one or more target tissue sites within brain 12 to manage patient symptoms associated with the patient disorder. Leads 20 may be implanted to position electrodes 24, 26 at desired locations of brain 12 through respective holes in cranium 32. Leads 20 may be placed at any location within brain 12 such that electrodes 24, 26 are capable of providing electrical stimulation to target tissue sites within brain 12 during treatment. In the example shown in FIG. 1, leads 20 are implanted within the right and left hemispheres, respectively, of brain 12 in order deliver electrical stimulation to one or more regions of brain 12, which may be selected based on many factors, such as the type of patient condition for which therapy system 10 is implemented to manage.

Different neurological or psychiatric disorders may be associated with activity in one or more of regions of brain 12, which may differ between patients. Thus, stimulation therapy may be delivered to different regions of brain 12 depending on the patient condition and symptoms of the patient condition. For example, in the case of MDD, bipolar disorder, OCD or other anxiety disorders, leads 20 may be implanted to deliver electrical stimulation to the anterior limb of the internal capsule of brain 12, and only the ventral portion of the anterior limb of the internal capsule (also referred to as a VC/VS), the subgenual component of the cingulate cortex, anterior cingulate cortex Brodmann areas 32 and 24, various parts of the prefrontal cortex, including the dorsal lateral and medial pre-frontal cortex (PFC) (e.g., Brodmann area 9), ventromedial prefrontal cortex (e.g., Brodmann area 10), the lateral and medial orbitofrontal cortex (e.g., Brodmann area 11), the medial or nucleus accumbens, thalamus, intralaminar thalamic nuclei, amygdala, hippocampus, the lateral hypothalamus, the Locus ceruleus, the dorsal raphe nucleus, ventral tegmentum, the substantia nigra, subthalamic nucleus, the inferior thalamic peduncle, the dorsal medial nucleus of the thalamus, the habenula, or any combination thereof

Suitable target therapy delivery sites within brain 20 for controlling a movement disorder of patient 14 include the pedunculopontine nucleus (PPN), thalamus, basal ganglia structures (e.g., globus pallidus, substantia nigra or subthalamic nucleus), zona inserta, fiber tracts, lenticular fasciculus (and branches thereof), ansa lenticularis, and/or the Field of Forel (thalamic fasciculus). The PPN may also be referred to as the pedunculopontine tegmental nucleus.

The target therapy delivery site may depend upon the patient disorder or condition being treated. Thus, in other examples, leads 20 may be positioned to deliver other types of therapy to patient 14, such as spinal cord stimulation to manage pain, proximate to a pelvic floor nerve to manage urinary or fecal incontinence, or proximate to any other suitable nerve, organ, muscle or muscle group in patient 14, which may be selected based on, for example, a patient condition. For example, therapy system 10 may be used to deliver neurostimulation therapy to a pudendal nerve, a perineal nerve or other areas of the nervous system, in which cases, one or both leads 20 would be implanted and substantially fixed proximate to the respective nerve. As further examples, one or both leads 20 may be positioned for temporary or chronic spinal cord stimulation for the treatment of pain, for peripheral neuropathy or post-operative pain mitigation, ilioinguinal nerve stimulation, intercostal nerve stimulation, gastric stimulation for the treatment of gastric mobility disorders and obesity, muscle stimulation (e.g., functional electrical stimulation (FES) of muscles), for mitigation of other peripheral and localized pain (e.g., leg pain or back pain), or for deep brain stimulation to treat movement disorders and other neurological disorders. Accordingly, although patient 14 and DBS are referenced throughout the remainder of the disclosure for purposes of illustration, a therapy system may be adapted for use in a variety of electrical stimulation applications.

Although leads 20 are shown in FIG. 1 as being coupled to a common lead extension 18, in other examples, leads 20 may be coupled to IMD 16 via separate lead extensions or directly coupled to connector block 30 of IMD 16. Leads 20 may deliver electrical stimulation to treat any number of neurological disorders or diseases, such as psychiatric disorders, movement disorders or seizure disorders. Examples of movement disorders include a reduction in muscle control, motion impairment or other movement problems, such as rigidity, bradykinesia, rhythmic hyperkinesia, nonrhythmic hyperkinesia, dystonia, tremor, and akinesia. Movement disorders may be associated with patient disease states, such as Parkinson's disease or Huntington's disease. An example seizure disorder includes epilepsy.

Leads 20 may be implanted within a desired location of brain 12 via any suitable technique, such as through respective burr holes in a skull of patient 14 or through a common burr hole in the cranium. Leads 20 may be placed at any location within brain 12 such that the electrodes of the leads are capable of providing electrical stimulation to targeted tissue during treatment. Electrical stimulation generated from the signal generator (not shown) within the therapy module of IMD 16 may help prevent the onset of events associated with the patient's condition or mitigate symptoms of the patient condition. The exact therapy parameter values of the stimulation therapy, such as the amplitude or magnitude of the stimulation signals, the duration of each signal, the waveform of the stimuli (e.g., rectangular, sinusoidal or ramped signals), the frequency of the signals, and the like, may be specific for the particular target stimulation site (e.g., the region of the brain) involved as well as the particular patient and patient condition.

In the case of stimulation pulses, the stimulation therapy may be characterized by selected pulse parameters, such as pulse amplitude, pulse rate, and pulse width. In addition, if different electrodes are available for delivery of stimulation, the therapy may be further characterized by different electrode combinations, which can include selected electrodes and their respective polarities. Known techniques for determining the optimal stimulation parameters may be employed.

The electrodes 24, 26 of leads 20 are shown as ring electrodes. Ring electrodes may be relatively easy to program and are typically capable of delivering an electrical field to any tissue adjacent to leads 20. In other examples, the electrodes of leads 20 may have different configurations. For example, the electrodes of leads 20 may have a complex electrode array geometry that is capable of producing shaped electrical fields. The complex electrode array geometry may include multiple electrodes (e.g., partial ring or segmented electrodes) around the perimeter of each lead 20, rather than a ring electrode. In this manner, electrical stimulation may be directed to a specific direction from leads 20 to enhance therapy efficacy and reduce possible adverse side effects from stimulating a large volume of tissue. In some examples, a housing of IMD 16 may include one or more stimulation and/or sensing electrodes. In alternative examples, leads 20 may have shapes other than elongated cylinders as shown in FIG. 1. For example, leads 20 may be paddle leads, spherical leads, bendable leads, or any other type of shape effective in treating patient 14.

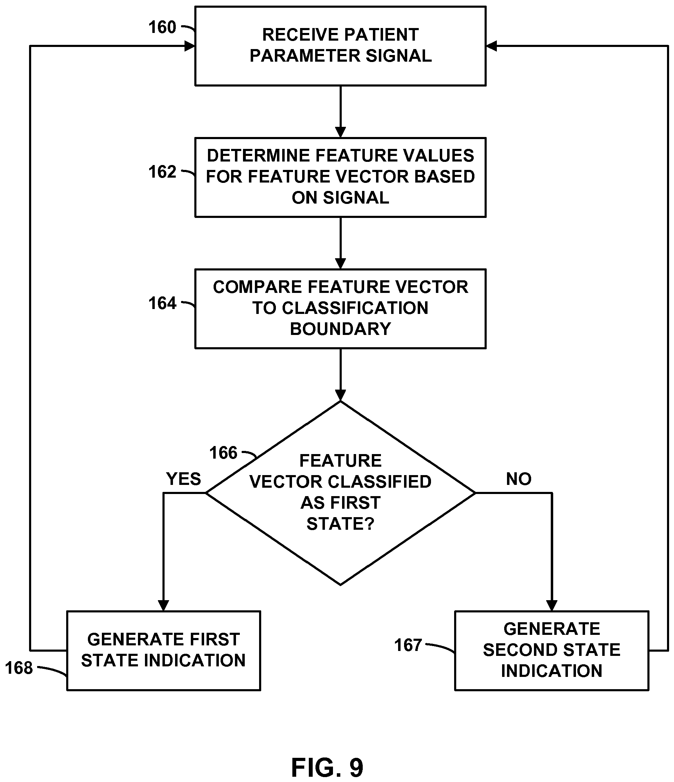

In some examples IMD 16 includes a sensing module that senses bioelectrical signals within brain 12 or communicates with a sensing module that is physically separate from IMD 16. The bioelectrical brain signals may reflect changes in electrical current produced by the sum of electrical potential differences across brain tissue. Examples of bioelectrical brain signals include, but are not limited to, an EEG signal, ECoG signal, a LFP sensed from within one or more regions of a patient's brain and/or action potentials from single cells within the patient's brain. In addition, in some cases, a bioelectrical brains signal includes a measured impedance of tissue of brain 12. In some examples, the bioelectrical brain signals may be used to determine whether patient 14 is in a particular state, e.g., using a classification boundary determined with a SVM algorithm, as described with reference to FIG. 9.

In some examples, leads 20 may include sensing electrodes positioned to detect the bioelectrical brain signal within one or more region of patient's brain 12. Alternatively, another set of implantable or external sensing electrodes may monitor the electrical signal. IMD 16 may deliver therapy and sense bioelectrical brain signals within the same or different target tissue sites of brain 12.

IMD 16 includes a stimulation generator that generates the electrical stimulation delivered to patient 14 via leads 20. In the example shown in FIG. 1, IMD 16 generates the electrical stimulation according to one or more therapy parameters, which may be arranged in a therapy program (or a parameter set). In particular, a signal generator (not shown) within IMD 16 produces the stimulation in the manner defined by the therapy program or group of programs selected by the clinician and/or patient 14. The signal generator may be configured to produce electrical pulses to treat patient 14. In other examples, the signal generator of IMD 16 may be configured to generate a continuous wave signal, e.g., a sine wave or triangle wave. In either case, IMD 16 generates the electrical stimulation therapy for DBS according to therapy parameter values defined by a particular therapy program.

A therapy program defines respective values for a number of parameters that define the stimulation. For example, the therapy parameters may include voltage or current pulse amplitudes, pulse widths, pulse rates, pulse frequencies, electrode combinations, and the like. IMD 16 may store a plurality of programs. In some cases, the one or more stimulation programs are organized into groups, and IMD 16 may deliver stimulation to patient 14 according to a program group. During a trial stage in which IMD 16 is evaluated to determine whether IMD 16 provides efficacious therapy to patient 14, the stored programs may be tested and evaluated for efficacy.

IMD 16 may include a memory to store one or more therapy programs (e.g., arranged in groups), and instructions defining the extent to which patient 14 may adjust therapy parameters, switch between programs, or undertake other therapy adjustments. Patient 14 may generate additional programs for use by IMD 16 via programmer 28 at any time during therapy or as designated by the clinician.

Generally, outer housing 34 of IMD 16 is constructed of a biocompatible material that resists corrosion and degradation from bodily fluids. IMD 16 may be implanted within a subcutaneous pocket close to the stimulation site. Although IMD 16 is implanted within a subcutaneous pocket near a clavicle of patient 14 in the example shown in FIG. 1, in other examples, IMD 16 may be implanted within cranium or at another tissue site (e.g., a submuscular tissue site or tissue site near an abdomen of patient 14). In addition, while IMD 16 is shown as implanted within patient 14 in FIG. 1, in other examples, IMD 16 may be located external to the patient. For example, IMD 16 may be a trial stimulator electrically coupled to leads 20 via a percutaneous lead during a trial period. If the trial stimulator indicates therapy system 10 provides effective treatment to patient 14, the clinician may implant a chronic stimulator within patient 14 for long-term treatment.

In some examples, depending on upon the patient condition, therapy system 10 includes motion sensor 36, which generates a signal indicative of patient activity (e.g., patient movement or patient posture transitions). For example, motion sensor 36 may include one or more accelerometers (e.g., one-axis, two-axis or three-axis accelerometers) capable of detecting static orientation or vectors in three-dimensions. An example accelerometer is a micro-electromechanical accelerometer. In other examples, motion sensor 36 may alternatively or additionally include one or more gyroscopes, pressure transducers, piezoelectric crystals, or other sensors that generate a signal that changes as a function of patient activity and patient posture. In some examples, the signal generated by motion sensor 36 may be used to determine whether patient 14 is in a particular state, e.g., using the SVM-based technique described with reference to FIG. 9 or another supervised machine learning technique implemented by a computing device.

In some examples, patient input provided via programmer 28 or IMD 16 may also be correlated with bioelectrical brain signal information or other signals indicative of a patient parameter in order to train a patient state detection algorithm (e.g., a SVM algorithm). For example, as described with respect to FIG. 4, the patient input may indicate when patient 14 is in a specific patient state, such as at least one of a seizure state, a particular movement disorder state, a mood state, a particular patient posture, or the like. Patient 14 may provide input via programmer 28 or IMD 16 (e.g., by tapping IMD 16 in a predetermined pattern, and IMD 16 may include a motion detector to detect the patient input) to indicate the patient state occurred. The input may also indicate a time at which the patient state occurred, such that the patient input may be temporally correlated with the bioelectrical brain signal information. One or more brain signal characteristics that are indicative of the patient state may be determined using, for example, the technique described with respect to FIG. 4.

In some examples, the patient input received via programmer 28 or another device can be used to activate recording of training data used by the SVM technique implemented by a computing device (e.g., programmer 28, IMD 16 or another computing device) to generate the SVM-based classification algorithm for patient state detection. In some examples, the training data includes a signal generated by a sensor (e.g., a motion sensor and/or physiological parameter sensing module), which can be stored in a memory of IMD 16 upon the receipt of patient input via programmer 28. The signal can be recorded for a predetermined length of time (e.g., about one minute or less) or until further patient input is received via programmer 28. In some examples, a memory of IMD 16 can buffer data that is sensed prior to the receipt of patient input. In such examples, the training data can include the signal generated by the sensor indicative of a patient parameter for a time period both prior to and after the receipt of the patient input that activated the recording of the training data. As discussed in further detail below, other techniques can be used to acquire training data in addition to or instead of the patient input.

Example systems and techniques for receiving patient input to collect information related to the occurrence of a patient event, such as a mood state or a seizure state, are described in U.S. Patent Application Serial No. 12/236,211 to Kovach et al., entitled, "PATIENT EVENT INFORMATION," which was filed on September 23, 2008. As described in U.S. Patent Application Serial No. 12/236,211 to Kovach et al., a processor of programmer 28 or another computing device may generate an event marker upon activation of an event indication button of programmer 28 by patient 14. For example, if patient 14 detects a seizure or a particular mood state or patient posture, patient 14 may activate the event indication button, and, in response, the processor may generate an event marker. Other types of patient events are contemplated, such as occurrences of other types of patient states (e.g., movement state, a particular mood state, a particular posture state, and the like). Patient 14 may provide event information relating to the patient event. For example, the event information may include the type of patient event detected, the severity of the patient event, duration of the patient event, drug type and dose taken prior to, during or after the occurrence of the patient event, a subjective rating of the efficacy of therapy that is delivered to manage the patient condition, and the like. Programmer 28 may provide a user interface that is configured to receive the event information from the patient, and, in some examples, may prompt the patient for the information.

In the example shown in FIG. 1, motion sensor 36 is located within outer housing 34 of IMD 16. In other examples, motion sensor 36 may be implanted at any suitable location within patient 14 or may be carried externally to patient 14. The location for motion sensor 36 may be selected based on various factors, such as the type of patient motion that motion sensor 36 is implemented to detect. Motion sensor 36 may be separate from IMD 16 in some examples. A motion sensor that is physically separate from IMD 16 or leads 20 may communicate with IMD 16 via wireless communication techniques or a wired connection. In some examples, therapy system 10 includes more than one motion sensor 36. For example, multiple implanted or external motion sensors may be positioned to detect movement of multiple limbs (e.g., arms or legs) of patient 14.

In some examples, therapy system 10 also include a sensor 38 that generates a signal indicative of a patient parameter in addition or instead of motion sensor 36 or a sensing module of IMD 16. Sensor 38 may be any suitable sensor that senses a physiological parameter associated with a patient condition of patient 14. Although shown as being physically separate from IMD 16 in the example shown in FIG. 1, in other examples, sensor 38 may be on or within an outer housing of IMD 16. Sensor 38 may be implanted within patient 14 at any suitable location (e.g., a subcutaneous implant site) or may be external (e.g., not implanted within patient 14).

In some examples, sensor 38 is configured to monitor a physiological signal of patient 14 such as a heart rate, respiratory rate, electrodermal activity (e.g., skin conductance level or galvanic skin response), muscle activity (e.g., via electromyogram), thermal sensing, and any other physiological parameter that may be indicative of a particular patient state. In some examples, however, a sensing module of IMD 16 may also sense one or more of these physiological parameters.

External programmer 28 wirelessly communicates with IMD 16 as needed to provide or retrieve therapy information. Programmer 28 is an external computing device that the user, e.g., the clinician and/or patient 14, may use to communicate with IMD 16. For example, programmer 28 may be a clinician programmer that the clinician uses to communicate with IMD 16 and program one or more therapy programs for IMD 16. Alternatively, programmer 28 may be a patient programmer that allows patient 14 to select programs and/or view and modify therapy parameters. The clinician programmer may include more programming features than the patient programmer. In other words, more complex or sensitive tasks may only be allowed by the clinician programmer to prevent an untrained patient from making undesired changes to IMD 16.

Programmer 28 may be a handheld computing device with a display viewable by the user and an interface for providing input to programmer 28 (i.e., a user input mechanism). For example, programmer 28 may include a small display screen (e.g., a liquid crystal display (LCD) or a light emitting diode (LED) display) that presents information to the user. In addition, programmer 28 may include a touch screen display, keypad, buttons, a peripheral pointing device or another input mechanism that allows the user to navigate though the user interface of programmer 28 and provide input. If programmer 28 includes buttons and a keypad, the buttons may be dedicated to performing a certain function, i.e., a power button, or the buttons and the keypad may be soft keys that change in function depending upon the section of the user interface currently viewed by the user. Alternatively, the screen (not shown) of programmer 28 may be a touch screen that allows the user to provide input directly to the user interface shown on the display. The user may use a stylus or their finger to provide input to the display.

In other examples, programmer 28 may be a larger workstation or a separate application within another multi-function device, rather than a dedicated computing device. For example, the multi-function device may be a notebook computer, tablet computer, workstation, cellular phone, personal digital assistant or another computing device that may run an application that enables the computing device to operate as a secure medical device programmer 28. A wireless adapter coupled to the computing device may enable secure communication between the computing device and IMD 16.

When programmer 28 is configured for use by the clinician, programmer 28 may be used to transmit initial programming information to IMD 16. This initial information may include hardware information, such as the type of leads 20, the arrangement of electrodes 24, 26 on leads 20, the number and location of motion sensor 36 within patient 14, the position of leads 20 within brain 12, the configuration of electrode array 24, 26, initial programs defining therapy parameter values, and any other information the clinician desires to program into IMD 16. Programmer 28 may also be capable of completing functional tests (e.g., measuring the impedance of electrodes 24, 26 of leads 20).

The clinician may also store therapy programs within IMD 16 with the aid of programmer 28. During a programming session, which may occur after implantation of IMD 16 or prior to implantation of IMD 16, the clinician may determine the therapy parameter values that provide efficacious therapy to patient 14 to address symptoms associated with the patient condition. For example, the clinician may select one or more electrode combinations with which stimulation is delivered to brain 12. As another example, programmer 28 or another computing device may utilize a search algorithm that automatically selects therapy programs for trialing, i.e., testing on patient 14. During the programming session, patient 14 may provide feedback to the clinician as to the efficacy of the specific program being evaluated (e.g., trialed or tested) or the clinician may evaluate the efficacy based on one or more physiological parameters of patient (e.g., heart rate, respiratory rate, or muscle activity). Programmer 28 may assist the clinician in the creation/identification of therapy programs by providing a methodical system for identifying potentially beneficial therapy parameter values.

Programmer 28 may also be configured for use by patient 14. When configured as a patient programmer, programmer 28 may have limited functionality (compared to a clinician programmer) in order to prevent patient 14 from altering critical functions of IMD 16 or applications that may be detrimental to patient 14. In this manner, programmer 28 may only allow patient 14 to adjust values for certain therapy parameters or set an available range of values for a particular therapy parameter.

Programmer 28 may also provide an indication to patient 14 when therapy is being delivered, when patient input has triggered a change in therapy or when the power source within programmer 28 or IMD 16 needs to be replaced or recharged. For example, programmer 28 may include an alert LED, may flash a message to patient 14 via a programmer display, generate an audible sound or somatosensory cue to confirm patient input was received, e.g., to indicate a patient state or to manually modify a therapy parameter.

Whether programmer 28 is configured for clinician or patient use, programmer 28 is configured to communicate to IMD 16 and, optionally, another computing device, via wireless communication. Programmer 28, for example, may communicate via wireless communication with IMD 16 using radio frequency (RF) telemetry techniques known in the art. Programmer 28 may also communicate with another programmer or computing device via a wired or wireless connection using any of a variety of local wireless communication techniques, such as RF communication according to the 802.11 or Bluetooth specification sets, infrared (IR) communication according to the IRDA specification set, or other standard or proprietary telemetry protocols. Programmer 28 may also communicate with other programming or computing devices via exchange of removable media, such as magnetic or optical disks, memory cards or memory sticks. Further, programmer 28 may communicate with IMD 16 and another programmer via remote telemetry techniques known in the art, communicating via a local area network (LAN), wide area network (WAN), public switched telephone network (PSTN), or cellular telephone network, for example.

Therapy system 10 may be implemented to provide chronic stimulation therapy to patient 14 over the course of several months or years. However, system 10 may also be employed on a trial basis to evaluate therapy before committing to full implantation. If implemented temporarily, some components of system 10 may not be implanted within patient 14. For example, patient 14 may be fitted with an external medical device, such as a trial stimulator, rather than IMD 16. The external medical device may be coupled to percutaneous leads or to implanted leads via a percutaneous extension. If the trial stimulator indicates DBS system 10 provides effective treatment to patient 14, the clinician may implant a chronic stimulator within patient 14 for relatively long-term treatment.

In addition to or instead of electrical stimulation therapy, IMD 16 may deliver a therapeutic agent to patient 14 to manage a patient condition in addition to or instead of electrical stimulation therapy. In such examples, IMD 16 may include a fluid pump or another device that delivers a therapeutic agent in some metered or other desired flow dosage to the therapy site within patient 14 from a reservoir within IMD 16 via a catheter. The fluid pump may be external or implanted. The therapeutic agent may be used to provide therapy to patient 14 to manage a condition of patient 14, and may be delivered to the patient's brain 12, blood stream or tissue. As another example, a medical device may be an external patch that is worn on a skin surface of patient 14, where the patch elutes a therapeutic agent, which is then absorbed by the patient's skin. Other types of therapeutic agent delivery systems are contemplated. IMD 16 may deliver the therapeutic agent upon detecting a particular patient state based on a signal indicative of a patient parameter (e.g., a bioelectrical brain signal or a motion sensor signal). The catheter used to deliver the therapeutic agent to patient 14 may include one or more electrodes for sensing bioelectrical brain signals of patient 14.

In the case of therapeutic agent delivery, the therapy parameters may include the dosage of the therapeutic agent (e.g., a bolus size or concentration), the rate of delivery of the therapeutic agent, the maximum acceptable dose in each bolus, a time interval at which a dose of the therapeutic agent may be delivered to a patient (lock-out interval), and so forth.

While the remainder of the disclosure describes various systems, devices, and techniques for detecting a patient state of patient 14 with respect to therapy system 10 of FIG. 1, the systems, devices, and techniques described herein are also applicable to other types of therapy systems, such as therapy systems that deliver a therapeutic agent to patient 14 to manage a patient condition or therapy systems that only provide a notification to patient 14 upon detection of a patient state. In some cases, the therapy system may be used for monitoring one or more signals indicative of one or more parameters of patient 14 and may not include therapy delivery (e.g., stimulation delivery or therapeutic agent delivery) capabilities. The monitoring device may be useful for the clinician during, for example, initial evaluation of patient 14 to evaluate the patient condition and the generation of a classification boundary for classifying a portion of a sensed patient parameter signal as indicative of a first patient state or a state other than the first state using a SVM algorithm, as described with reference to FIG. 4.

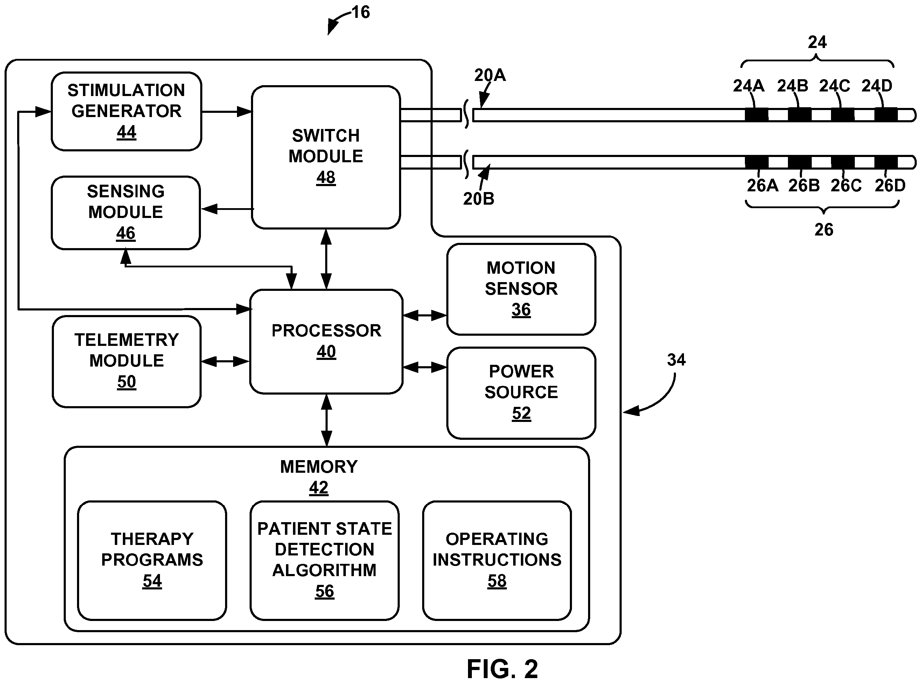

FIG. 2 is a functional block diagram illustrating components of an example IMD 16 in greater detail. In the example shown in FIG. 2, IMD 16 includes motion sensor 36, processor 40, memory 42, stimulation generator 44, sensing module 46, switch module 48, telemetry module 50, and power source 52. Memory 42 may include any volatile or non-volatile media, such as a random access memory (RAM), read only memory (ROM), non-volatile RAM (NVRAM), electrically erasable programmable ROM (EEPROM), flash memory, and the like. Memory 42 may store computer-readable instructions that, when executed by processor 40, cause IMD 16 to perform various functions described herein. In addition, in some examples, memory 42 store data generated by motion sensor 36 and/or sensing module 46 for training the SVM to generate a classification boundary for the SVM-based algorithm.

In the example shown in FIG. 2, memory 42 stores therapy programs 54, patient state detection algorithm 56, and operating instructions 58 in separate memories within memory 42 or separate areas within memory 42. Each stored therapy program 54 defines a particular program of therapy in terms of respective values for electrical stimulation parameters, such as a stimulation electrode combination, electrode polarity, current or voltage amplitude, and, in if stimulation generator 44 generates and delivers stimulation pulses, the therapy programs may define values for a pulse width, pulse rate, and duty cycle of a stimulation signal. In some examples, the therapy programs may be stored as a therapy group, which defines a set of therapy programs with which stimulation may be generated. The stimulation signals defined by the therapy programs of the therapy group may be delivered together on an overlapping or non-overlapping (e.g., time-interleaved) basis.

Patient state detection algorithm 56 stored by memory 42 includes machine-readable instructions for performing an algorithm. Using the instructions, processor 40 may execute patient state detection algorithm 56 to detect a patient state based on a received signal that is indicative of a patient parameter (e.g., a signal from sensing module 46, motion sensor 36 or sensor 38 shown in FIG. 1). An example patient state detection algorithm with which processor 40 may detect a patient state uses a classification boundary generated with a SVM. An example of this patient state detection technique is described with respect to FIG. 9. Operating instructions 58 guide general operation of IMD 16 under control of processor 40, and may include instructions for, e.g., measuring the impedance of electrodes 24, 26 and/or determining the distance between electrodes 24, 26.

In some examples, memory 42 also stores a log (or record) of patient state occurrences. This may be useful for evaluating the patient condition, the progression of the patient condition, or the therapeutic effects of IMD 16 in managing the patient condition. The log of patient state occurrences can include any suitable type of information. In one example, the log includes a patient state indication generated by processor 40 upon the detection of the patient state, a date and time stamp indicating when the patient state was detected, and the patient parameter signal generated by any one or more of motion sensor 36, sensor 28, sensing module 46, or another sensing module.

IMD 16 is coupled to leads 20A and 20B, which include electrodes 24A-24D and 26A-26D, respectively (collectively "electrodes 24 and 26"). Although IMD 16 is coupled directly to leads 20, in other examples, IMD 16 may be coupled to leads 20 indirectly, e.g., via lead extension 18 (FIG. 1). In the example shown in FIG. 2, implantable medical leads 20 are substantially cylindrical, such that electrodes 24, 26 are positioned on a rounded outer surface of leads 20. As previously described, in other examples, leads 20 may be, at least in part, paddle-shaped (i.e., a "paddle" lead). In some examples, electrodes 24, 26 may be ring electrodes. In other examples, electrodes 24, 26 may be segmented or partial ring electrodes, each of which extends along an arc less than 360 degrees (e.g., 90-120 degrees) around the outer perimeter of the respective lead 20. The use of segmented or partial ring electrodes 24, 26 may also reduce the overall power delivered to electrodes 24, 26 by IMD 16 because of the ability to more efficiently deliver stimulation to a target stimulation site by eliminating or minimizing the delivery of stimulation to unwanted or unnecessary regions within patient 14.

The configuration, type, and number of electrodes 24, 26 illustrated in FIG. 2 are merely exemplary. For example, IMD 16 may be coupled to one lead with eight electrodes on the lead or three or more leads with the aid of bifurcated lead extensions. Electrodes 24, 26 are electrically coupled to stimulation generator 44 and sensing module 46 of IMD 16 via conductors within the respective leads 20A, 20B. Each of electrodes 24, 26 may be coupled to separate conductors so that electrodes 24, 26 may be individually selected, or in some examples, two or more electrodes 24 and/or two or more electrodes 26 may be coupled to a common conductor. In some examples, sensing module 46 senses bioelectrical brain signals via electrodes selected from electrodes 24, 26, although other electrodes or sensing device may also be used.

Processor 40 may include any one or more of a microprocessor, a controller, a digital signal processor (DSP), an application specific integrated circuit (ASIC), a field-programmable gate array (FPGA), discrete logic circuitry. The functions attributed to processors described herein may be embodied in a hardware device via software, firmware, hardware or any combination thereof. Processor 40 controls the stimulation generator 44 to generate and deliver electrical stimulation signals to patient 14 according to selected therapy parameters. Specifically, processor 40 controls stimulation generator 44 according to therapy programs 54 stored in memory 42 to apply particular stimulation parameter values specified by one or more programs, such as current or voltage amplitude, frequency, and duty cycle (e.g., pulse width and pulse rate in the case of stimulation pulses). In addition, processor 40 may also control stimulation generator 44 to deliver the electrical stimulation signals via selected subsets of electrodes 24, 26 with selected polarities. For example, switch module 48 may combine electrodes 24, 26 in various bipolar or multi-polar combinations to deliver stimulation energy to selected sites, such as sites within brain 12. In other examples, therapy programs are stored within programmer 28 or another computing device, which transmits the therapy programs to IMD 16 via telemetry module 50.

In the example shown in FIG. 2, the set of electrodes 24 of lead 20A includes electrodes 24A, 24B, 24C, and 24D, and the set of electrodes 26 of lead 20B includes electrodes 26A, 26B, 26C, and 26D. Processor 40 may control switch module 48 to apply the stimulation signals generated by stimulation generator 44 to selected combinations of electrodes 24, 26. In particular, switch module 48 may couple stimulation signals to selected conductors within leads 20, which, in turn, deliver the stimulation signals across selected electrodes 24, 26. Switch module 48 may be a switch array, switch matrix, multiplexer, or any other type of switching module configured to selectively couple stimulation energy to selected electrodes 24, 26 and to selectively sense bioelectrical brain signals with selected electrodes 24, 26. Hence, stimulation generator 44 is coupled to electrodes 24, 26 via switch module 48 and conductors within leads 20. In some examples, however, IMD 16 does not include switch module 48.

Stimulation generator 44 may be a single channel or multi-channel stimulation generator. In particular, stimulation generator 44 may be capable of delivering, a single stimulation pulse, multiple stimulation pulses or continuous signal at a given time via a single electrode combination or multiple stimulation pulses at a given time via multiple electrode combinations. In some examples, however, stimulation generator 44 and switch module 48 may be configured to deliver multiple channels on a time-interleaved basis. For example, switch module 48 may serve to time divide the output of stimulation generator 44 across different electrode combinations at different times to deliver multiple programs or channels of stimulation energy to patient 14.

Sensing module 46 is configured to sense bioelectrical brain signals of patient 14 via a selected subset of electrodes 24, 26. Processor 40 may control switch module 48 to electrically connect sensing module 46 to selected combinations of electrodes 24, 26. In this way, sensing module 46 may selectively sense bioelectrical brain signals with different combinations of electrodes 24, 26. As previously described, in some examples, processor 40 may detect a particular patient state of patient 14 via the sensed bioelectrical brain signal. In other examples, processor 40 may detect a particular patient state of patient 14 based on other physiological parameters of patient 14 in addition to or instead of a bioelectrical brain signal indicative of brain activity.

In some examples, sensing module 46 includes a frequency selective sensing circuit that extracts the energy level within one or more selected frequency bands of a sensed patient parameter signal, which may be, for example, a bioelectrical brain signal. The frequency selective sensing circuit can include a chopper-stabilized superheterodyne instrumentation amplifier and a signal analysis unit, and may utilize a heterodyning, chopper-stabilized amplifier architecture to convert a selected frequency band of a physiological signal, such as a bioelectrical brain signal, to a baseband for analysis. The physiological signal may be analyzed in one or more selected frequency bands to determine one or more features as described herein. In some examples, sensing module 46 includes a plurality of channels that extract the same or different frequency bands of one or more signals indicative of one or more patient parameters.

Examples of various additional chopper amplifier circuits that may be suitable for or adapted to the techniques, circuits and devices of this disclosure are described in U.S. Patent No. 7,385,443 to Denison, which is entitled "CHOPPER STABILIZED INSTRUMENTATION AMPLIFIER" and issued on January 10, 2008. Examples of frequency selective monitors that may utilize a heterodyning, chopper-stabilized amplifier architecture are described in U.S. Provisional Application No. 60/975,372 by Denison et al., entitled "FREQUENCY SELECTIVE MONITORING OF PHYSIOLOGICAL SIGNALS," and filed on September 26, 2007, commonly-assigned U.S. Provisional Application No. 61/025,503 by Denison et al., entitled "FREQUENCY SELECTIVE MONITORING OF PHYSIOLOGICAL SIGNALS, and filed on February 1, 2008, and commonly-assigned U.S. Provisional Application No. 61/083,381, entitled, "FREQUENCY SELECTIVE EEG SENSING CIRCUITRY," and filed on July 24, 2008. Further examples of chopper amplifier circuits are also described in further detail in commonly-assigned U.S. Patent Application Publication No. 2009/0082691 by Denison et al., entitled, "FREQUENCY SELECTIVE MONITORING OF PHYSIOLOGICAL SIGNALS" and filed on September 25, 2008.

A sensing module 46 that directly extracts energy in key frequency bands of a bioelectrical brain signal may be used to extract bandpower at key physiological frequencies with an architecture that is flexible, robust, and relatively low-noise. Chopper stabilization is a noise and power efficient architecture for amplifying low-frequency neural signals in micropower applications (e.g., an implanted device) with excellent process immunity. Chopper stabilized amplifiers can be adapted to provide wide dynamic range, high-Q filters. A sensing module 46 that includes a chopper-stabilized amplifier may slightly displace the clocks within the chopper amplifier in order to re-center a targeted band of energy to direct current (DC) in a manner similar to superheterodyne receivers used in communication systems. In some examples, extracting the bandpower within a selected frequency band requires two parallel signal paths (in-phase and quadrature) that are combined within the power extraction stage. The power output signal can be lowpass filtered, which results in an output that represents the spectral power fluctuations in the frequency band.

As previously indicated, a bioelectrical brain signal may include an EEG, ECoG, single cell recording, or LFP. The band power fluctuations in LFPs sensed within brain 12 of patient 14 (FIG. 1) are generally orders of magnitude slower than the frequency at which they are encoded, so the use of efficient analog preprocessing before performing analog to digital conversion can greatly reduce the overall energy requirements for implementing a complete mixed-signal system. Thus, a sensing module 46 that includes a circuit architecture that directly extracts energy in key frequency bands of a bioelectrical brain signal may be useful for tracking the relatively slow power fluctuations within the selected frequency bands and determining a patient state based on the bioelectrical brain signal. In some examples, the energy in particular frequency band or bands of a bioelectrical brain signal may be used as a parameter that serves as a feature value in a supervised learning algorithm, such as an SVM algorithm or an SVM-based classification algorithm generated based on the SVM algorithm. An example of such a sensing module 46 is a chopper-stabilized superheterodyne instrumentation amplifier and a signal analysis unit.

In the example shown in FIG. 2, IMD 16 includes motion sensor 36, which is enclosed with a common housing with processor 40, stimulation generator 44, and sensing module 46. As previously described, in other examples, motion sensor 36 is connected to a lead and/or implanted separately from IMD 16 within patient 14, or may be external to patient 14. Motion sensor 36 may comprise any suitable device that generates an electrical signal that is indicative of patient motion or patient posture. For example, motion sensor 36 may comprise a single axis, 2-axis or 3-axis accelerometer, a piezoelectric crystal, a gyroscope, a pressure transducer or any combination of accelerometers, piezoelectric crystals, gyroscopes or pressure transducers. Signals from motion sensor 36 are provided to processor 40, which may detect a patient state based on the signal, e.g., using a classification boundary determined using a SVM algorithm, e.g., as described with respect to FIG. 9.

Telemetry module 50 supports wireless communication between IMD 16 and an external programmer 28 or another computing device under the control of processor 40. Processor 40 of IMD 16 may receive, as updates to programs, values for various stimulation parameters such as amplitude and electrode combination, from programmer 28 via telemetry module 50. The updates to the therapy programs may be stored within therapy programs 54 portion of memory 42. Telemetry module 50 in IMD 16, as well as telemetry modules in other devices and systems described herein, such as programmer 28, may accomplish communication by radiofrequency (RF) communication techniques. In addition, telemetry module 50 may communicate with external medical device programmer 28 via proximal inductive interaction of IMD 16 with programmer 28. Accordingly, telemetry module 50 may send information to external programmer 28 on a continuous basis, at periodic intervals, or upon request from IMD 16 or programmer 28.

Power source 52 delivers operating power to various components of IMD 16. Power source 52 may include a small rechargeable or non-rechargeable battery and a power generation circuit to produce the operating power. Recharging may be accomplished through proximal inductive interaction between an external charger and an inductive charging coil within IMD 16. In some examples, power requirements may be small enough to allow IMD 16 to utilize patient motion and implement a kinetic energy-scavenging device to trickle charge a rechargeable battery. In other examples, traditional batteries may be used for a limited period of time.

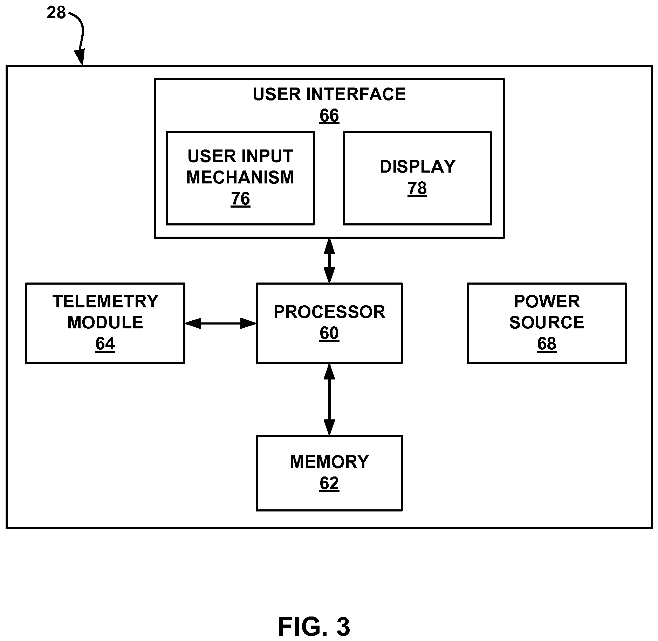

FIG. 3 is a conceptual block diagram of an example external medical device programmer 28, which includes processor 60, memory 62, telemetry module 64, user interface 66, and power source 68. Processor 60 controls user interface 66 and telemetry module 64, and stores and retrieves information and instructions to and from memory 62. Programmer 28 may be configured for use as a clinician programmer or a patient programmer. Processor 60 may comprise any combination of one or more processors including one or more microprocessors, DSPs, ASICs, FPGAs, or other equivalent integrated or discrete logic circuitry. Accordingly, processor 60 may include any suitable structure, whether in hardware, software, firmware, or any combination thereof, to perform the functions ascribed herein to processor 60.

A user, such as a clinician or patient 14, may interact with programmer 28 through user interface 66. User interface 66 includes user input mechanism 76 and display 78, such as a LCD or LED display or other type of screen, to present information related to the therapy, such as information related to bioelectrical signals sensed via a plurality of sense electrode combinations. Display 78 may also be used to present a visual alert to patient 14 that IMD 16 has detected a particular patient state is about to occur. Other types of alerts are contemplated, such as audible alerts or somatosensory alerts. Input mechanism 76 is configured to receive input from the user. Input mechanism 76 may include, for example, buttons, a keypad (e.g., an alphanumeric keypad), a peripheral pointing device or another input mechanism that allows the user to navigate though user interfaces presented by processor 60 of programmer 28 and provide input.

Input mechanism 76 can include buttons and a keypad, where the buttons may be dedicated to performing a certain function, i.e., a power button, or the buttons and the keypad may be soft keys that change function depending upon the section of the user interface currently viewed by the user. Alternatively, display 78 of programmer 28 may be a touch screen that allows the user to provide input directly to the user interface shown on the display. The user may use a stylus or their finger to provide input to the display. In other examples, user interface 66 also includes audio circuitry for providing audible instructions or notifications to patient 14 and/or receiving voice commands from patient 14, which may be useful if patient 14 has limited motor functions. Patient 14, a clinician or another user may also interact with programmer 28 to manually select therapy programs, generate new therapy programs, modify therapy programs through individual or global adjustments, and transmit the new programs to IMD 16.

In some examples, at least some of the control of therapy delivery by IMD 16 may be implemented by processor 60 of programmer 28. For example, in some examples, processor 60 may receive patient activity information and bioelectrical brain signals from IMD 16 or from a sensing module that is separate from IMD 16. The separate sensing module may, but need not be, implanted within patient 14. In some examples, processor 60 may evaluate the patient activity information and bioelectrical brain signals from IMD 16 to determine which of a plurality of patient states patient 14 is currently in.

In addition, in some examples, instead of or in addition to processor 40 of IMD 16 or another computing device, processor 60 of programmer 28 may generate one or more boundaries using a SVM algorithm for determining a patient state based on a sensed patient parameter signal. An example technique that processor 60 can implement in order to train the SVM algorithm (or another supervised machine learning algorithm) to determine the one or more boundaries is described with respect to FIG. 4.

Memory 62 may include instructions for operating user interface 66 and telemetry module 64, and for managing power source 68. Memory 62 may also store any therapy data retrieved from IMD 16 during the course of therapy, as well as instructions for a SVM that may be implemented to generate a classification boundary for detecting patient states. Memory 62 may include any volatile or nonvolatile memory, such as RAM, ROM, EEPROM or flash memory. Memory 62 may also include a removable memory portion that may be used to provide memory updates or increases in memory capacities. A removable memory may also allow sensitive patient data to be removed before programmer 28 is used by a different patient. In some examples, memory 62 can also store a log of patient state detections, as described above with respect to memory 42 of IMD 16.

Wireless telemetry in programmer 28 may be accomplished by RF communication or proximal inductive interaction of external programmer 28 with IMD 16. This wireless communication is possible through the use of telemetry module 64. Accordingly, telemetry module 64 may be similar to the telemetry module contained within IMD 16. In alternative examples, programmer 28 may be capable of infrared communication or direct communication through a wired connection. In this manner, other external devices may be capable of communicating with programmer 28 without needing to establish a secure wireless connection.

Power source 68 delivers operating power to the components of programmer 28. Power source 68 may include a battery and a power generation circuit to produce the operating power. In some examples, the battery may be rechargeable to allow extended operation. Recharging may be accomplished by electrically coupling power source 68 to a cradle or plug that is connected to an alternating current (AC) outlet. In addition, recharging may be accomplished through proximal inductive interaction between an external charger and an inductive charging coil within programmer 28. In other examples, traditional batteries (e.g., nickel cadmium or lithium ion batteries) may be used. In addition, programmer 28 may be directly coupled to an alternating current outlet to operate. Power source 68 may include circuitry to monitor power remaining within a battery. In this manner, user interface 66 may provide a current battery level indicator or low battery level indicator when the battery needs to be replaced or recharged. In some cases, power source 68 may be capable of estimating the remaining time of operation using the current battery.

In some examples, programmer 28 implements the SVM-based classification algorithm (or another supervised machine learning based classification algorithm) in order to determine a patient state. In such examples, memory 62 stores a patient state detection algorithm similar to patient state detection algorithm 56 stored by memory 42 of IMD 16. The patient state detection algorithm stored by memory 62 can include machine-readable instructions for performing an algorithm. Using the instructions, processor 60 of programmer 28 may execute the patient state detection algorithm to detect a patient state based on a received signal that is indicative of a patient parameter. Processor 60 can receive the signal from sensing module 46, motion sensor 36, sensor 38 or another sensor via wired or wireless communication techniques.

In other examples, a computing device that is remotely located from IMD 16 and programmer 28 (e.g., at a clinician's office) can implements the SVM-based classification algorithm (or another supervised machine learning based classification algorithm) in order to determine a patient state. As with programmer 28, the remote computing device can receive a patient parameter signal from sensing module 46, motion sensor 36, sensor 38 or another sensor via wired or wireless communication techniques. The signal can be transmitted to the remote computer continuously or periodically. However, depending on the available bandwidth for the transmission of signals from IMD 16 or another sensing module to programmer 28 or a remote computer, it may be desirable for IMD 16 (or the other sensing module) to transmit parameterized signals or data rather than raw signal waveforms.

A SVM technique is a supervised machine learning technique used for classification and regression that views input data as sets of vectors in an n-dimensional space (also referred to as a feature space). The feature space may have any suitable number of dimensions, such as two, three, four or more. A SVM-based algorithm (also referred to herein as an "SVM algorithm") classifies data segments, such as characteristics (or "features") of a signal indicative of a patient parameter, as indicative of different patient states. The SVM algorithm learns how to classify data segments based on representative feature values that are indicative of patient 14 being in a first patient state and representative feature values that are indicative of patient 14 not being in the first patient state (e.g., indicative of a second patient state). As previously indicated, a feature value may be a value indicative of a characteristic of a patient parameter signal (e.g., morphology of the signal or the spectral characteristics of the signal), and a feature vector includes respective values for each of a plurality of features. The patient parameter signal may be a bioelectrical brain signal, as primarily described herein, or may be another type of signal indicative of a patient parameter, such as a signal from motion sensor 36 (also referred to as a posture sensor or an activity sensor), sensor 38 (FIG. 1) or sensing module 46 (FIG. 2). The techniques described herein for determining feature vectors and classifying patient states based on a bioelectrical brain signal are also applicable to other types of patient parameter signals.

Feature values are associated with a particular patient state. As discussed above, a feature vector includes respective values for each of a plurality of features (e.g., two or more features) for a segment of a patient parameter signal. A computing device (e.g., programmer 28, IMD 16 or another computing device) executing the SVM algorithm defines a classification boundary based on a plurality of feature vectors, where the classification boundary separates a feature space into two different regions. Each feature of the feature space defines an axis, such that the values of the feature vector indicate the coordinates of a point within the feature space. That is, a feature vector can be mapped to a specific point within a feature space based on the values of the features in the feature vector.

The known feature values (also referred to as representative feature values) are determined based on training data (e.g., data associating a signal indicative of a physiological parameter or patient posture state with a particular patient state). The training data can be acquired using any suitable technique. In some examples, as described above, IMD 16 or programmer 28 records and stores a sensor signal and an indication of an occurrence of a patient state temporally associated with the recorded physiological signal. In some examples, the sensor signal can be stored in a loop recorder, although other memory formats are also contemplated. The sensor signal recording and storing can be initiated using any suitable technique. Various examples are described with respect to FIG. 4. An example loop recording technique is described in commonly assigned U.S. Patent No. 7,610,083 by Drew et al., which is entitled, "METHOD AND SYSTEM FOR LOOP RECORDING WITH OVERLAPPING EVENTS" and issued on October 27, 2009.

A clinician can later evaluate the recorded training data (e.g., sensor data and data indicating occurrences of one or more patient states) to determine the representative feature values for each of one or more patient states. In other examples, the representative feature values are provided by a user (e.g., a clinician) input during a learning stage, which may be prior to implementation of therapy by IMD 16 or during a follow-up session in which the patient detection algorithm of IMD 16 is updated. The representative feature values can be specific to a particular patient 14 or may be based on training data that is general to more than one patient.

The clinician may select two or more features that are useful for identifying the first and second patient states based on a patient parameter signal, as well as determine the feature vector values (e.g., with the aid of a computing device), which are then inputted into the SVM algorithm. Feature values determined based on a segment of a patient parameter signal are arranged in a vector, which is referred to as a feature vector, which is mapped to the feature space, which may be two-dimensional, three-dimensional, or have any other number of dimensions.

Based on the representative feature vectors, the SVM algorithm generates a classification boundary (also referred to as a hyperplane in the case of a linear boundary) in the feature space. The classification boundary separates the feature space into a first region associated with feature values indicative of the first patient state and a second region associated with feature values indicative of the second patient state. The classification boundary can be a two-dimensional boundary or can extend in more than two directions.

A SVM algorithm generates a classification boundary for patient state detection based on the feature values that are determined based on a sensed patient parameter signal for a particular patient 14. In this way, the SVM can be trained based on data specific to patient 14 such that the classification boundary implemented by a device at later time to detect the patient state is generated based on patient-specific data.

In some existing techniques for detecting a patient state, a patient state is determined by comparing one or more signal characteristics to a threshold value or template that is not specific to the patient, but is applied to multiple patients. The signal characteristic can be, for example, an amplitude of a physiological signal, one or more power levels in the frequency domain of the physiological signal, or a pattern in the physiological signal waveform. While detecting the patient state based on a non-patient specific threshold value or template may be useful, the number of false positive patient state detections and false negative patient state detections may be higher compared to techniques in which patient-specific classification boundaries are used to detect a patient state. A SVM-based classification algorithm is configured to improve patient state detection compared to some existing techniques because the SVM-based classification algorithm is generated using an SVM that relies on patient-specific training data and generates a classification boundary for a particular patient.

Some patient parameter signal characteristics that are indicative of a patient state may be similar for a class of patients, and, therefore, the non-patient specific threshold value or template can be useful for detecting the patient state. However, the techniques for detecting a patient state that rely on a non-patient specific threshold value or signal template do not necessarily consider the ways in which the patient parameters may differ between patients. These differences in patient parameters between patients may result in different sensitivities and specificities of patient state detection algorithms for different patients despite the use of the same patient state detection threshold value or template.

As an example, a first patient with an anxiety disorder may have a relatively high power level in a particular frequency band of a bioelectrical brain signal when the first patient is not in an anxious state (i.e., is in an non-anxious state), whereas a second patient with a similar anxiety disorder may have a lower power level in the particular frequency band of a bioelectrical brain signal when in a non-anxious state compared to the first patient. Thus, the biomarkers indicative of the non-anxious states of the first and second patients may differ. A non-patient specific threshold value may not account for these differences, and may, for example, result in the mischaracterization of some non-anxious states of the first patient as an anxious state because of the higher overall power level in the particular frequency band during a non-anxious state.

The SVM and the resulting SVM-based classification algorithm that is used herein to used to distinguish between two different patient states accounts for differences in patient parameters between patients. In particular, the SVM is trained to automatically classify a patient state based on actual patient parameter data for a specific patient 14, where the patient parameter data is known to be indicative of a first patient state. In some examples, the SVM is also trained based on actual patient parameter data for a specific patient 14 that is known to be indicative of a second patient state that is not the first patient state. The SVM-based classification algorithm for different patients may, therefore, define different classification boundaries with which a computing device determines a patient state.

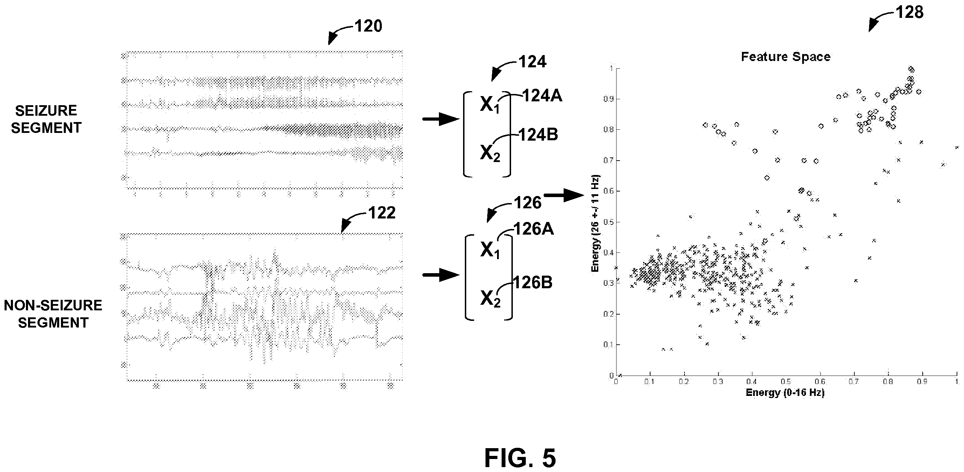

FIG. 4 is a flow diagram of an example technique for training a SVM (also referred to as a SVM algorithm) to respond to future patient parameter signal inputs and classify the patient parameter signal inputs as being representative of the first patient state or a second patient state. A SVM can generate a classification boundary used by IMD 16 or another device at a later time to determine whether a sensed patient parameter signal is indicative of a particular patient state using the technique shown in FIG. 4. The technique shown in FIG. 4 may be performed by IMD 16, programmer 28 or another computing device. Thus, while processor 60 of programmer 28 is referred to throughout the description of FIG. 4, as well as FIGS. 6-8 and processor 40 of IMD 16 is referred to throughout the description of FIGS. 5 and 9-19, in other examples, any part of the techniques described herein may be implemented by processor 40 of IMD 16 (FIG. 2), processor 60 of programmer 28, a processor of another medical device (e.g., an external medical device), another computing device, or a combination thereof.

In accordance with the technique shown in FIG. 4, processor 60 receives an indication of a first patient state (100), which may be, for example, a patient mood state, a movement state, posture state or any of the other patient states discussed above. In some examples, patient 14 provides input indicating the occurrence of the patient state via user interface 66 (FIG. 3) of programmer 28 or another user input mechanism, such as a device dedicated to receiving input from patient 14 indicative of the occurrence of the patient state. The dedicated device can be, for example, a key fob with a limited number of input buttons (e.g., one or two buttons), a consumer electronic device (e.g., a cell phone or a personal digital assistant) that is configured to record the patient inputs, or any other suitable device capable of receiving and storing patient input. Processor 60 may receive the input from the dedicated device through a wired (e.g., a cable) connection or via a wireless connection.

In other examples, processor 60 can automatically determine the occurrence of the patient state based on data from a sensor alone or in combination with patient input. The SVM-based algorithm can be implemented in order to permit processor 60 to automatically detect a patient state based on a signal from a first type of sensor. Processor 60 can automatically determine a patient state based on a signal from a second type of sensor, which can be, for example, a sensor that is reliable for patient state detection, but is not useful for ambulatory IMD control because of its size, power consumption or other factors. Hence, the second type of sensor can be used to train processor 60 to detect a patient state based on the first type of sensor.

The indication of the first patient state may include a date and time stamp to indicate the time at which the first patient state was detected or the time at which patient 14 provided input indicating the occurrence of the first patient state. Depending upon the condition (e.g., a disorder) with which patient 14 is diagnosed, patient 14 may be unable to provide input indicating the occurrence of the first patient state until after the onset of the first patient state, and even after the termination of the first patient state. Thus, programmer 28 may include features that permit patient 14 to modify the patient input, such as by modifying the date and time stamp associated with the patient input to be more accurate. In some examples, patient 14 may also provide input indicating the end of the patient state.

IMD 16 may receive direct patient input in some examples. For example, patient 14 may tap the skin superior to IMD 16 and IMD 16 may include a motion sensor that is configured to sense a particular pattern of tapping, which is then characterized as patient input.

Processor 60 also receives a signal indicative of a patient parameter (102). In some examples, processor 60 receives the signal from IMD 16 or a separate implanted or external sensing device, either of which may generate a signal indicative of a physiological parameter (e.g., bioelectrical brain signals, heart rate, body temperature, and the like) or a signal indicative of another patient parameter, such as patient activity level or patient posture state. In some examples, processor 60 receives more than one signal indicative of a respective patient parameter.

In the examples described herein, processor 60 receives the signal from IMD 16. However, in other examples, processor 60 may receive the patient parameter signal from another sensing device instead or in addition to IMD 16. Moreover, in examples in which processor 40 of IMD 16 performs at least a part of the technique shown in FIG. 4, processor 40 may receive the signal from sensing module 46 (FIG. 2). In the example shown in FIG. 4, the signal is stored by IMD 16 or a separate sensing device, and processor 60 receives the signal from IMD 16 or the sensing device via wireless communication techniques. In examples in which IMD 16 comprises an external device, processor 60 may receive the signal from IMD 16 via a wired (e.g., a cable) connection. Processor 60 can receive the signal indicative of the patient parameter from IMD 16 on a substantially continuous basis, on a regular, periodic basis or processor 60 of programmer 28 may interrogate IMD 16 to retrieve the signal.

IMD 16 or the separate sensing device may sense the patient parameter on a continuous basis, a substantially periodic and scheduled basis, or in response to receiving patient input or another trigger. For example, upon receiving patient input via programmer 28 or directly via IMD 16, IMD 16 may begin storing the signal indicative of the patient parameter, and, in some examples, may also store the portion of the signal preceding the receipt of the patient input for at least a predetermined amount of time. IMD 16 may include a loop recorder or another type of memory to store the patient parameter signal, from which processor 40 of IMD 16 may retrieve the portion of the signal preceding the receipt of the patient input for storage in memory 42.

In some examples, processor 60 initiates the recording and storing of the sensor signal generated by motion sensor 36, sensor 38 or sensing module 46 in response to and immediately upon receiving patient input via user interface 66 (FIG. 3) of programmer 28 or another device indicating the occurrence of a particular patient state. In other examples, a generic algorithm can be used to trigger recording of the data. The generic algorithm may be, for example, an algorithm that generally detects the occurrence of the patient state, but with less precision and accuracy than the SVM based algorithm described herein. For example, the generic algorithm may be over-inclusive and provide more false positive detections of the patient state than the SVM based algorithm derived from the training data.

In one generic, patient-non-specific algorithm, motion sensor 36, sensor 38 or sensing module 46 generates a signal indicative of a patient parameter (e.g., posture, activity level or a physiological parameter) and extracts a spectral feature of the signal. A processor of IMD 16, programmer 28 or another device normalizes the sensed signal, such as by determining a ratio of the current energy to the background energy in a particular frequency band of the signal. The current energy level (e.g., a foreground energy level) in a particular frequency band can be determined based on a relatively short segment of the sensed signal (e.g., about 2 seconds), while the background energy can be determined based a longer time window of the sensed signal (e.g., about 30 minutes). According to the patient-non-specific algorithm, the processor determines that the patient state occurs when a ratio of the current energy to the background energy in a particular frequency band of the signal is greater than or equal to a predetermined threshold value. An example of the generic algorithm for predicting a change in an activity state of a patient's brain, which can indicate the occurrence of a patient state, is described in U.S. Patent No. 5,995,868 by Dorfmeister et al., which is entitled "SYSTEM FOR THE PREDICTION, RAPID DETECTION, WARNING, PREVENTION, OR CONTROL OF CHANGES IN ACTIVITY STATES IN THE BRAIN OF A SUBJECT," which issued on November 30, 1999.

In other examples, a timer controls when processor 60 initiates the recording and storing of the sensor signal generated by motion sensor 36, sensor 38 or sensing module 46. The duration of the timer can be set to activate data recording at predetermined time intervals or during different segments of the circadian cycle of patient 14. Recording sensor data from different segments of the circadian cycle of patient 14 may be useful for various patient conditions that exhibit different symptoms at different times during a day. For example, with respect to seizure disorders such as epilepsy, a brain signal (e.g., a LFP) during a non-ictal sleep state of patient 14 may differ from a brain signal during a non-ictal awake state of patient 14. The variations in the sensor signal during the different times of day may be useful for defining a precise and accurate classification boundary via the SVM.

In some examples, processor 60 initiates the recording and storing of the sensor signal generated by motion sensor 36, sensor 38 or sensing module 46 in response to the detection of a particular patient condition or event. The patient condition or event may be a surrogate marker for the patient state. For example, with respect to a patient diagnosed Major Depressive Disorder, motion sensor 36 can detect a depressive episode by detecting a time at which patient 14 exhibits a relatively low level of activity (e.g., as indicated by a predetermined threshold value or range) and processor 60 can initiate the recording of sensor data from at least one sensing module 38, 46 that senses a brain signal (e.g., an EEG, ECoG or LFP) upon the detection of the depressive episode in order to acquire brain signals that may be revealing of the depressive episode. As another example, with respect to a patient diagnosed with a seizure disorder, it may be useful to initiate recording of training data from one or more sensors 36, 38, 46 upon the onset of a seizure or a particular type of seizure. An onset of a seizure or a particular type of seizure can be automatically determined using any suitable technique, such as based on an analysis of data generated by motion sensor 36 or via an intracranial pressure sensor.

As described in commonly-assigned U.S. Patent Application Publication No. 12/359,055 by Giftakis et al., which is entitled "SEIZURE DISORDER EVALUATION BASED ON INTRACRANIAL PRESSURE AND PATIENT MOTION" and was filed on January 23, 2009, and commonly-assigned U.S. Patent Application Publication No. 12/359,037 by Giftakis et al., which is entitled "SEIZURE DISORDER EVALUATION BASED ON INTRACRANIAL PRESSURE" and was filed on January 23, 2009, patient motion and/or intracranial pressure can be used to detect an occurrence of a seizure state. In addition, seizure metrics can be generated based on intracranial pressure and/or patient motion associated with seizures. The seizure metrics can be used to assess a patient's seizures and distinguish between different types of seizures. For example, a type of seizure or a severity of the seizure may be determined based on a detected activity level of the patient during a seizure. In addition, a sudden change in patient posture during a time that corresponds to a detected seizure may indicate the patient fell during the seizure, which can indicate a relatively severe seizure that merits the recording of training data for purposes of determining a classification boundary for identifying future patient states in which such seizures are likely to occur.

In each of these examples of data recording triggers, the sensor data can be recorded for a predetermined length of time following the receipt of the trigger by processor 60 or processor 40 of IMD 16. As described above, memory 42 of IMD 16, memory 62 of programmer 28 or a memory of another device can also buffer data that was recorded prior to the receipt of any of the aforementioned triggers in order to obtain sensor signals for a time period prior to the patient-indicated occurrence of the patient state. As described in U.S. Patent No. 7,610,083 by Drew et al., an implantable medical device can store loop recordings of waveform data having specified pre-event and post-event times. The event can be indicated by, for example, the trigger.

After receiving the indications of the patient state and the patient parameter signal (100, 102), processor 60, automatically or with the aid of a clinician, identifies portions of the signal that are indicative of the first patient state (104). In some examples, processor 60 may temporally correlate the patient parameter signal with the indications of the first patient state to determine which portions of the patient parameter signal were sensed during the first patient state. In addition, in some examples, processor 60 also identifies the portions of the patient parameter signal that temporally correlate with the time immediately preceding the onset of the patient state and immediately after the termination of the patient state. Processor 60 may identify the portion of the patient parameter signal indicative of the first patient state as the portion that corresponds to a predetermined range of time prior to the indication of the occurrence of the first patient state and a predetermined range of time after the occurrence of the patient state, if such information is known.

Processor 60 also identifies portions of the patient parameter signal that are indicative of patient 14 being in a state other than the first state, i.e., indicative of patient 14 being in the second state (104). In general, the second state may be a specific patient state (e.g., a manic state) or may generally be a state that is not the first state. The SVM classifies data segments as indicating the first state or not. Thus, the second state can generally be a state that is not the first state.

In other examples, processor 60 identifies the signal portions indicative of the first and second patient states (104) based on input from the clinician. The clinician may determine which segments of a sensed patient parameter signal are associated with the first patient state and input the information to processor 60. In some examples in which the recording of data from at least one sensor 36, 38, 46 is triggered based on the receipt of an indication of an occurrence of a patient state from a user (e.g., patient 14, a patient caretaker or a clinician), processor 60 may not need to identify portions of the signal that are indicative of the patient state. Instead, the entire stored data segment may be associated with the patient state indicated by patient 14 or the automatically detected patient state.

After identifying the relevant portions of the patient parameter signal indicative of the first and second patient states (104), processor 60, automatically or with the aid of a clinician, determines feature vectors based on the identified portions of the patient parameter signal (106). A feature vector is a vector defined by two or more feature values indicative of a patient parameter signal characteristic (e.g., a morphology of the signal). In some examples, at least one of the features of the feature vector includes morphological features such as the power level (also referred to as spectral energy) of the patient parameter signal in one or more frequency bands, an amplitude (e.g., the instantaneous, peak, mean or median amplitude) of the portion of the patient parameter signal or a subportion of the portion, other signal characteristics, or combinations thereof.

A feature vector can include any number of features of the identified portion of the patient parameter signal. In some examples described herein, the feature vector includes two features. For example, if the first patient state is a seizure state and the second patient state is a non-seizure state, one feature may be the power level in the patient parameter signal portion in a frequency band of about 0 Hz to about 16 Hz, and another feature may be the power level in the signal portion in a frequency band of about 15 Hz to about 37 Hz.

The features of the feature vectors are be selected to help distinguish between the different patient states. In some examples, a clinician selects the features by evaluating the signal portions indicative of the first and second patient states and determining which signal characteristics help distinguish between the patient states. In other examples, processor 60 automatically determines the features of the feature vectors. In general, processor 60 selects the features such that the values of features associated with the first patient state differ significantly from the values of the features associated with the second patient state (e.g., a specific patient state or a general state other than the first patient state), such that the features of a sensor signal can be used to classify a patient state with accuracy and precision.

In examples in which the features are different frequency bands, the specific frequency bands that exhibit different power levels in the first and second states may not be known in advance of implementing the SVM. Accordingly, during the acquisition of the training data, IMD 16 or programmer 28 (or another device) can record the time-domain sensor signal, which is broadband data and includes a full spectrum. The clinician or processor 60 can perform an analysis at a later time to determine which sensing channels and features result in a significant (e.g., maximum) separation boundary of the first and second patient states. Each sensing channel of sensing module 46 of IMD 16 or another sensing module can extract a respective frequency band of a sensed patient parameter signal. In some examples, processor 60 presents a plurality of features that result in a significant (e.g., maximum) separation boundary of the first and second patient states to a clinician via display 78 (FIG. 3) and the clinician can select the features via user input mechanism 78.

In some examples, the clinician can select the features by simulating the classification boundary that results from the feature vectors that include the selected features. For example, after receiving user input indicating one or more selected features (e.g., different frequency bands) via user input mechanism 76 of programmer 28 (FIG. 3), processor 60 can generate a classification boundary based on the selected features and present a graphical display of the classification boundary, feature space, and feature vectors that include the feature vectors to the clinician via display 78 (FIG. 3). In this way, the clinician can visually analyze a plurality of classification boundaries and select the features that result in a classification boundary that appears to provide a relatively significant separation (e.g., as indicated by distance) between the different feature vectors associated with each of the two patient states delineated by the classification boundary.

In examples in which processor 60 automatically determines the features, processor 60 can implement a search algorithm to determine which frequency bands or other signal characteristics are revealing of the first and second patient states. When implementing the search algorithm, processor 60 can scan through the different combinations of sensing channels and frequency bands, determine classification boundary using any suitable technique such as the techniques described below, and generates a separation metric for each combination. The separation metric can indicate, for example, the mean, median, greatest or smallest distance between the classification boundary and the training feature values determined based on the training data and used to generate the classification boundary. In general, a greater distance between a training feature value and the classification boundary indicates that the features used to generate the classification boundary provide a better separation between the first and second patient states. Processor 60 can then present the one or more features associated with the greatest separation metrics to the clinician via display 78 of user interface 66 (FIG. 3). Processor 60 can also generate separation metrics based on combinations of sensing channels and frequency bands selected by a clinician, rather than generating separation metrics for combinations of sensing channels and frequency bands selected by processor 60 as described above.

After selecting the sensing channels of sensing module 46 or another sensor (e.g., sensor 38) that sensed a signal particularly revealing of the patient states, sensing module 46 can be configured to sense via selected sensing channels. In addition, after determining the frequency bands that are revealing of a particular patient state, sensing module 46 can be tuned to sense in the selected frequency bands.

It may be desirable to limit the number of features used by the SVM because of limitations of the sensing capabilities of IMD 16 or the power consumption limits of IMD 16. In other examples, the feature vector can include up to 16 or more features. For example, the feature vector can include the power level in ten separate frequency bands. If IMD 16 includes sixteen separate channels for sensing, each channel can be used to extract any number of features for a respective feature vector. For example, for each channel, the energy in each of 10 separate energy bands could be used define the respective feature vector.

Each feature in the feature vector corresponds to one dimension in the feature space that the SVM uses to classify data segments as being representative of the first patient state or a second patient state (e.g., a state that is generally different than the first patient state or a specific, known state). Each feature vector defines a point in a feature space with that the SVM implemented by a computing device uses to classify data. In this way, each data point defined by a feature vector is a quantitative representation of the monitored feature values for a given time and each feature vector defines one data point in the feature space that is used to generate the classification boundary. A feature vector may include any suitable number of features, such as two, three or more, and, accordingly, a feature space may have any suitable number of dimensions.

In some examples, processor 60 automatically determines the feature vectors (106), e.g., by automatically determining the values of each of the selected features for each of the identified signal portions. In other examples, a clinician or another person determines the feature vectors and inputs the determined feature values of the feature vectors into programmer 28 for automatic determination of the classification boundary.

In some examples, the signal portions on which each feature vector is determined has a predetermined duration of time. As a result, each feature vector represents the patient state for that predetermined duration of time. Accordingly, a single occurrence of a patient state that persists for a period of time that is longer than the duration of the signal portion used to determine a single feature vector may be associated with multiple feature vectors. In some examples, the signal segment used to determine a feature vector has a duration of about 0.5 seconds to about 5 seconds, such as about 2 seconds, although other time windows are contemplated.

An example of a technique in which a patient parameter signal is used to determine representative feature vectors, which provide training points for defining a classification boundary is shown in FIG. 5. FIG. 5 is a conceptual illustration of a supervised learning technique for configuring a SVM to generate a classification boundary for classifying a sensed patient parameter signal as indicative of a first state or a second state. In FIG. 5, IMD 16 senses a first bioelectrical brain signal segment 120 (also referred to as a portion of a signal) indicative of a seizure state of patient 14 and a second bioelectrical brain signal segment 122 indicative of a state that is not the seizure state.

Multiple frequency band components of the signals 120, 122 are shown in FIG. 5. In some examples, sensing module 46 of IMD 16 includes an analog sensing circuit with an amplifier that uses limited power to monitor a frequency in which a desired biosignal is generated. As previously indicated, the frequency selective sensing circuit can include a chopper-stabilized superheterodyne instrumentation amplifier and a signal analysis unit, and may utilize a heterodyning, chopper-stabilized amplifier architecture to convert a selected frequency band of a physiological signal, such as a bioelectrical brain signal, to a baseband for analysis. The physiological signal may be analyzed in one or more selected frequency bands to determine one or more features as described herein.

In the example shown in FIG. 5, sensing module 46 extracts particular frequency bands of the respective bioelectrical brain signals 120, 122 as features of the signals, such that the spectral energy in selected frequency bands can be determined to generate the respective feature vectors 124, 126. Processor 40 may sample and digitize signals 120, 122 at a relatively slow rate, such as a rate of about 1 Hz, when using the frequency selective sensing circuit. The relatively slow rate can be used because the sensing amplifier of sensing module 46 has already extracted the desired spectral energy features.

Processor 40 determines feature vector 124 based on sensed signal 120, where the feature value 124A of feature vector 124 is the energy level within a first frequency band of about 0 Hz to about 16 Hz, and second feature value 124B is the energy level within a second frequency band of about 15 Hz to about 37 Hz. Other frequency bands are contemplated and may be selected based on, for example, the frequency bands that are believed to be particularly revealing of the first and second patient states. In addition, feature vectors including more than two features are contemplated.

Processor 40 also determines feature vector 126 based on sensed signal 122, where feature value 126A of feature vector 126 is the energy level within a first frequency band of about 0 Hz to about 16 Hz, and feature value 126B is the energy level within a second frequency band of about 15 Hz to about 37 Hz. Each feature vector 124, 126 defines a point in feature space 128, which the SVM algorithm uses to generate a classification boundary. Thus, in the example shown in FIG. 5, each of the feature vectors defines one data point in the feature space. As previously indicated, each feature in the feature vector corresponds to one dimension in the feature space. Thus, in the example shown in FIG. 5, a two-dimensional feature space 128 is shown.

Returning now to the technique shown in FIG. 4, after determining the feature vector for the identified signals portions (106), processor 60 determines whether there are additional indications of the first and second patient states for which the feature vectors have not been determined (108). If there are additional indications of the first patient state for which processor 60 has not determined the feature vectors, processor 60 may identify the relevant portions of the patient parameter signal associated with the respective indications of the first and second patient states (104) and determine the feature vectors associated with the respective indications of the first and second patient states (106) until no additional training points (e.g., feature vectors in the example shown in FIG. 4) are left to be determined. For example, if there is no additional training data available, processor 60 can discontinue determining training points.

Processor 60, automatically without user input or based on user input, determines the feature vectors for each of the identified signal portions (106). Thus, the feature vector values for both signal portions indicative of the first patient state and signal portion indicative of the second patient state are determined. In this way, the SVM algorithm implemented by processor 60 is trained to classify data based on known feature vectors that are associated with one of the first or second states. As shown in the example feature space 128 of FIG. 5, the feature vectors define a point in feature space 128. In the example shown in FIG. 5, each feature vector that corresponds to a detection of a seizure state (i.e., the first state in the example shown in FIG. 5) is plotted in feature space 128 as a circular mark and each feature vector that does not correspond to an occurrence of a seizure (i.e., the second state in the example shown in FIG. 5) is shown as an "X."

Each detection of the seizure state shown in feature space 128 is not necessarily a separate occurrence of a seizure. Instead, some seizure state detections indicated by a feature vector may be a segment of a common seizure occurrence and, in some examples, these seizure segments can be clustered together to detect a seizure. The concept of clustering neurological activity to detect and monitor the occurrence of neurological events (e.g., a seizure) is described in commonly assigned U.S. Patent No. 7,280,867 to Frei et al., which is entitled "CLUSTERING OF RECORDED PATIENT NEUROLOGICAL ACTIVITY TO DETERMINE LENGTH OF A NEUROLOGICAL EVENT" and issued on October 9, 2007.

Feature vectors are determined based on a portion of a sensed patient parameter signal. Thus, a single occurrence of a patient state that takes place over a period of time that is longer than the duration of the signal portion used to determine a single feature vector may be associated with multiple feature vectors.

After determining a plurality of feature vectors for the first and second states, processor 60 automatically determines the classification boundary delineating the first and second patient states based on the plurality of determined feature vectors (110). In particular, the classification boundary is defined to separate feature values associated with known patient states such that the feature values for a first patient state are on one side of the boundary and feature values from the second patient state are on the other. In this way, processor 60 separates the determined feature values (which may be arranged into feature vectors) into two classes, whereby a first class corresponds to the occurrence of the first patient state and the second class corresponds to the occurrence of the second patient state. The technique shown in FIG. 4 may be used during a training stage in which the training data is from a specific patient and the support vector machine is trained based on that data for the specific patient. In this way, the patient-specific classification boundary may reduce the number of false positive and false negative patient state detections. In general, as the similarity between the patient states for which the classification boundary is used to differentiate increases, more support vectors may be needed to define a more complex classification boundary.

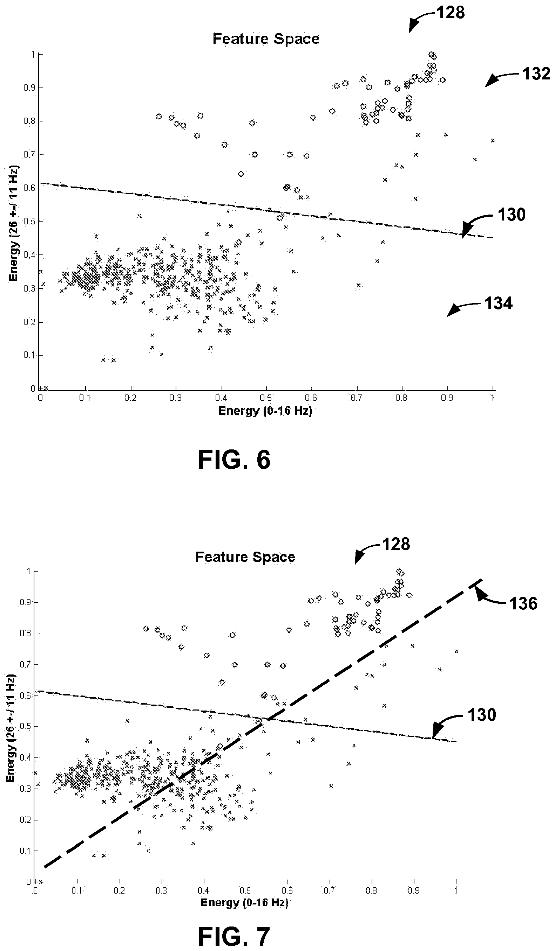

The classification boundary may be linear or non-linear. An example of a linear classification boundary 130 is shown in FIG. 6. Linear boundary 130 defines first region 132 and second region 134 of feature space 128, which are later used by the SVM to classify a sensed patient state based on a sensed patient parameter signal. First region 132 is associated with the first patient class, which, in the example shown, in FIG. 6 is a seizure state. Second region 134 is associated with the second patient class, which, in the example shown in FIG. 6, is a non-seizure state. Processor 60 automatically determines linear boundary 130 to maximize separation between the first and second patient classes.

Any suitable technique for determining linear boundary 130 may be used. In some examples, processor 60 utilizes the following equation to determine a linear boundary 130:

$$W^T X + \beta > 0 \quad \text{(Equation 1)}$$

The variable "W" is a support vector, the variable "X" is a vector defined by each feature value of the known data points (i.e., the training feature vectors) in feature space 128, and "$\beta$" is a bias. The variable "T" indicates that the support vector is transposed. The vector W and bias term $\beta$ are parameters determined by the SVM learning algorithm.

In some examples, processor 40 may determine more than one linear boundary, such as two or more. FIG. 7 is a conceptual illustration of feature space 128 for which processor 40 has determined two linear boundaries 130, 136 to delineate the first and second classes of known data points, which correspond to first and second patient states. At a later time, when processor 40 of IMD 16 is determining whether patient 14 is in a first state or a second state based on a sensed patient parameter signal, processor 40 may run simultaneous linear SVMs and perform a logical operation (e.g., AND or OR) based on linear boundaries 130, 136 to determine the patient state that is indicated by the sensed patient parameter signal.

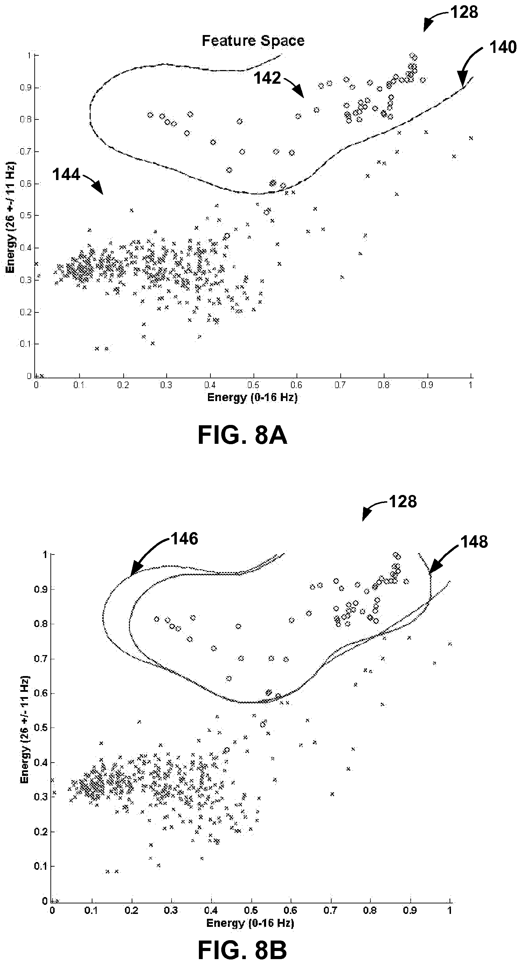

For example, processor 40 of IMD 16 may determine whether a feature vector extracted from a patient parameter signal indicates patient 14 is in a first state or a second state by simultaneously or consecutively determining whether the feature vector is classified as indicative of the first state or the second state based on linear boundary 130, and determining whether the feature vector is classified as indicative of the first state or the second state based on linear boundary 136. Utilizing linear SVMs with a plurality of linear boundaries 130, 136 results in a classification technique that is closer to a nonlinear SVM technique, which is described with respect to FIG. 8A. Utilizing a plurality of linear boundaries 130, 136, however, may require less processing by a processor compared to a SVM with a nonlinear boundary, and, therefore, may consume less power to classify patient 14 as being in a first patient state or a second patient state compared to a SVM that uses a nonlinear boundary.

An example nonlinear boundary 140 is shown in FIG. 8A. Nonlinear boundary 140 separates feature space 128 into first region 142 associated with a first patient state and second region 144 associated with the second patient state. As with the linear boundary, processor 60 determines the boundary 140 that maximizes separation between the first and second patient classes. Processor 60 may determine nonlinear boundary 140 based on the training data points (determined based on the feature vectors associated with the known first and second patient states) using any suitable technique. Processor 60 may, for example, use a kernel function to determine nonlinear boundary 140 that separates data points by patient state.

Processor 60 may utilize the following equation to determine a nonlinear classification boundary:

$$\beta + \sum_{i=1}^{N} \alpha_i \exp\left(-\gamma \left\| X - X_i \right\|^2\right) > 0 \quad \text{(Equation 2)}$$

In Equation 2, the variable "$\beta$" is a bias term, "$\alpha$" is a coefficient that is automatically determined by the SVM learning algorithm, "exp" indicates the following portion of the equation is an exponential of the coefficient "$\alpha$", the variable "$\gamma$" is user-defined to control the curve of the classification boundary and may be user-selected, and the variable "X" is a vector defined by each feature vector of the known data points (i.e., the training feature vectors) in feature space 128. In some examples, the variable $\gamma$ can be about 0.1. "$X_i$" indicates the representative support vectors that the SVM algorithm selects to define the curved boundary. Only some of the representative feature vectors are used to define the boundary, and the selected feature vectors may be referred to as support vectors.

A nonlinear boundary may provide a better separation of the first and second classes based on the training data points, but a processor may consume more power and time processing data segments to classify the data segments into the first and second classes using a nonlinear boundary. Power consumption may be an important factor when selecting a classification technique for an implantable medical device, such as IMD 16, because the useful life of IMD 16 may depend on the life of power source 52 (FIG. 2).

Determining nonlinear boundary 140 may also require more power consumption by processor 60 compared to determining linear boundary 130. It has been found that a processor may determine a nonlinear boundary that balances power consumption and specificity by limiting the number of terms of the exponential function of Equation 2. For example, it has been found that a nonlinear boundary generated with the eight terms (e.g., 8 support vectors) of the exponential function of Equation 2 generates an acceptable nonlinear boundary with a classification specificity that is close to the classification specificity resulting from a SVM with a nonlinear boundary generated with approximately 50 to approximately 200 terms of the exponential function of Equation 2. Thus, limiting the number of terms used to determine nonlinear boundary 132 in feature space 128 can make the use of a SVM that utilizes a nonlinear boundary more feasible for a device with limited processing capabilities and limited power sources, such as IMD 16. Classification specificity can be a function of the number of incorrect state detections, the number of false positive first state detections, and/or the number of false negative first state detections by the SVM.

FIG. 8B is a conceptual illustration of feature space 128 that compares nonlinear boundary 146 determined using the Equation 2 with eight terms and nonlinear boundary 148 determined using Equation 2 with 50 terms. As FIG. 8B shows, nonlinear boundary 146 determined using fewer terms is similar to boundary 148, and, therefore, may have a similar classification specificity. FIG. 8B suggests that the ability to generate a useful nonlinear boundary with a fewer number of terms may help limit the power consumption by processor 40 of IMD 16 when classifying a particular patient state.

After processor 60 automatically determines the classification boundary (block 110 in FIG. 4), the classification boundary generated using the SVM is loaded into a device that detects the patient states. For example, programmer 28, alone or with the aid of a clinician, may load the SVM into memory 42 (FIG. 2) of IMD 16. After this step, processor 40 of IMD 16 automatically processes a real-time or stored patient parameter signal and the SVM classifies a plurality of data segments extracted from the signal (e.g., a sample of the signal) using the determined classification boundary. In the examples described herein, the data segments are feature vectors determined based on the characteristics of the patient parameter signal. The SVM maps the feature vector determined based on the patient parameter signal sensed by IMD 16 into the feature space and determines which side of the classification boundary the vector feature lies. Based on this determination, the processor 40 determines a current patient state.

FIG. 9 is a flow diagram illustrating an example technique for determining a patient state based on a real-time or stored patient parameter signal with a classification boundary that was determined using a SVM algorithm. FIG. 9 is described with respect to processor 40 of IMD 16. However, the technique shown in FIG. 9 may be performed by processor 60 of programmer 28, a processor of another device or any combination thereof.

Processor 40 receives a signal indicative of a patient parameter (160). The signal can be, for example, a physiological signal or a signal indicative of patient activity level or patient posture. In some examples, the patient parameter signal that the SVM uses to determine the classification boundary is the same signal with which processor 40 determines the patient state. In some examples, the patient parameter signal is generated by sensing module 46 (FIG. 2), motion sensor 36, another sensor, or combinations thereof.

Processor 40 determines one or more feature values for determining a feature vector based on the signal (162). The features for which the values are determined are the same features with which the SVM algorithm generated the classification boundary, e.g., using the technique described in FIG. 4. Processor 40 can determine the feature vector values using any suitable technique, such as the technique described with respect to FIG. 4 for determining feature vectors for SVM training points. In some examples, processor 40 determines the feature vector based on a sample of the patient parameter signal having a predetermined duration of time. In this way, a plurality of determined feature vectors including respective feature values may represent the patient state for a known duration of time.

After determining the feature vector values (162) based on the received signal, processor 40 compares the feature vector values to a classification boundary (164), which may be linear (e.g., linear boundary 130 in FIG. 5) or nonlinear (e.g., nonlinear boundary 140 in FIG. 7). In particular, processor 40 maps the determined feature vector to the feature space and determines the side of the boundary in which the feature vector lies. In some examples, processor 40 is interested in determining whether patient 14 is in a first state. Thus, if the feature vector does not lie within a side of the boundary associated with the first patient state, processor 40 may generate a second state indication (167) and then continue monitoring a physiological signal (160) and determining the feature vector (162). The second state indication may be, for example, a value, flag or signal that is stored in memory 42 of IMD 16 or another device (e.g., programmer 28).

In other examples, processor 40 does not generate a second state indication, but merely continues monitoring a physiological signal (160) and determining the feature vector values (162) until the first state is detected. If the feature vector lies within a side of the boundary associated with the first patient state, processor 40 classifies the determined feature vector in the feature space associated with the first state and processor 40 determines that patient 14 is in the first state (166). Processor 40 may generate a first state indication (168). The first state indication may be, for example, a value, flag or signal that is stored in memory 42 of IMD 16 or another device (e.g., programmer 28). In some examples, processor 40 determines whether a predetermined number (e.g., four) of consecutive points are on one side of the boundary before determining patient 14 has changed states.

As previously indicated, determination of the first patient state may be used for various purposes, such as to control therapy delivery (e.g., initiate, deactivate or modify one or more parameters of therapy delivery), generate a patient notification (e.g., an alert to indicate that a seizure is about to occur), to evaluate a patient condition, or initiate recording of a patient parameter (and storing the patient parameter, such as a signal indicative of the patient parameter, in a memory of a device). Thus, upon generation of the first state indication (168), processor 40 of IMD 16 may take any suitable course of action, which may be preselected by a clinician and can include any one or more of the aforementioned actions.

In examples in which processor 40 of IMD 16 controls a therapy module (e.g., stimulation generator 44 (FIG. 2) in examples in which IMD 16 generates and delivers electrical stimulation to patient 14, a fluid delivery module in examples in which IMD 16 generates and delivers a therapeutic agent to patient 14 or an module that delivers an external cue) based on a determined patient state, processor 40 can modify one or more parameters of therapy delivery in response to the patient state determination. The modification (or adjustment) to the one or more therapy parameters differs from deactivation of therapy delivery in response to a detected patient state in the sense that IMD 16 continues to actively deliver therapy to patient 14 with the adjusted therapy parameters, rather than deactivates therapy delivery. In this way, IMD 16 can adjust therapy delivery to accommodate different patient states, which may be associated with different symptoms or different therapeutic results. This responsive therapy delivery helps provide efficacious therapy to patient 14.

In one example, processor 40 selects a therapy program from memory 42 (FIG. 2) or adjusts one or more stimulation parameter values for a current program (including parameters such as amplitude, pulse width, pulse rate, electrode combination, electrode polarity) based on a determined patient state. IMD 16 then generates and delivers therapy to patient according to the adjust therapy parameters. In examples in which IMD 16 delivers a therapeutic agent to patient 14 instead of or in addition to electrical stimulation, processor 40 can select a therapy program from memory 42 (FIG. 2) or adjust one or more fluid delivery parameter values (e.g., dosage of the therapeutic agent, a rate of delivery of the therapeutic agent, a maximum acceptable dose in each bolus, or a time interval at which a dose of the therapeutic agent may be delivered to a patient). Thereafter, IMD 16 delivers the therapeutic agent to patient 14 according to the adjusted parameters. In examples in an external device delivers an external cue to patient 14, such as a visual, auditory or somatosensory cue (e.g., a pulsed vibration), processor 40 of IMD 16 or a processor of another device, such as the external device, can control the external device to decrease or increase the contrast or brightness of a visual cue, increase or decrease the longevity of the visual cue, increase or decrease the volume of an auditory cue, and so forth.

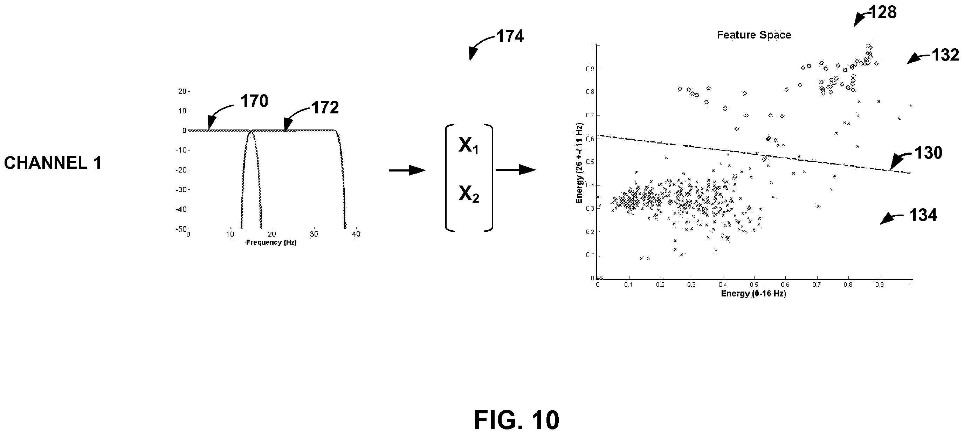

FIG. 10 is a conceptual illustration of the technique with which processor 40, while implementing a SVM algorithm, determines a patient state based on a signal indicative of a patient parameter. In FIG. 10, sensing module 46 of IMD 16 senses a bioelectrical brain signal of patient 14 with one sensing channel (CHANNEL 1). In the example shown in FIG. 10, sensing module 46 includes an analog frequency selective sensing circuit that extracts frequency components of bioelectrical signals sensed via the sensing channel. From the patient parameter signal sensed via CHANNEL 1, sensing module 46 extract values for a first feature 170 comprising the energy level in the frequency band of about 0 Hz to about 16 Hz, and a second feature 172 comprising the energy level in the frequency band of about 15 Hz to about 37 Hz. The values of these features 170, 172 are the feature values $X_1$ and $X_2$ of feature vector 174 generated for the sensing channels.

After determining the feature vector 174 with the feature values ($X_1$ and $X_2$), processor 40 maps the feature vector 174 to a previously determined feature space 128 (e.g., determined using the technique shown in FIG. 4) and determines the side of linear boundary 130 on which feature vector 174 lies. In other examples, the SVM algorithm may utilize a nonlinear boundary instead of or in addition to linear boundary 130. If feature vector 174 lies within region 132, processor 40 determines that the sensed bioelectrical brain signals indicate patient 14 is in a first state (e.g., a seizure state). On the other hand, if feature vector 174 maps to region 134, processor 40 determines that the sensed bioelectrical brain signals indicate patient 14 is in a second state (e.g., a non-seizure state) or at least is not in the first state.

Processor 40 determines whether patient 14 is in a first state or a second state with the aid of a classification boundary determined using a SVM algorithm. Processor 40 may determine whether patient 14 is in one of a plurality of patient states by utilizing a plurality of classification boundaries determined by a SVM algorithm, where each of the classification boundaries is used to determine whether patient 14 is in a respective state or not in the state.

In some examples, processor 40 of IMD 16 (or a processor of another device) may determine whether a sensed patient parameter signal indicates that patient 14 is moving towards the patient state for which a course of action is desirable. As previously indicated, the course of action can include delivery of therapy (e.g., stimulation or a pharmaceutical agent), delivery of a patient notification, initiation of recording of a patient parameter signal, and the like. Rather than waiting until the patient state is actually detected based on the patient parameter signal, processor 40 may initiate the course of action when the feature vectors determined based on the sensed patient parameter signal over a period of time indicate that patient 14 is moving towards the patient state.

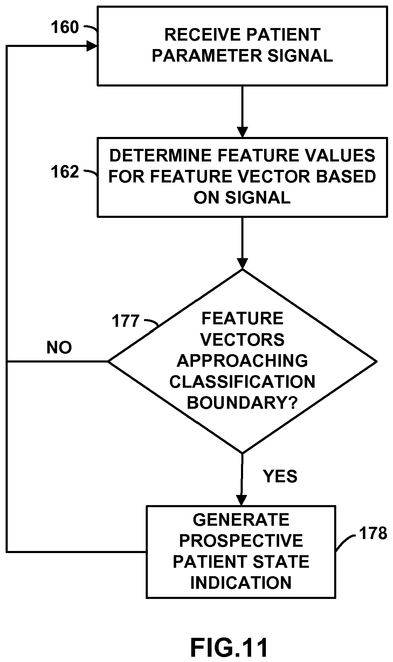

FIG. 11 is a flow diagram of an example technique for determining whether a sensed patient parameter signal indicates that patient 14 is moving towards a specific patient state. As with the technique shown in FIG. 9, processor 40 receives a signal indicative of a patient parameter (160) and determines one or more feature values for determining a feature vector based on a time segment of the signal (162). Processor 40 may determine a plurality of feature vectors based on respective portions of a sensed patient parameter signal over time, such that each feature vector indicates the patient state for a predetermined period of time. Feature vectors determined based on sequential (or consecutive) segments of the patient parameter signal may indicate sequential patient state determinations.

As previously discussed, the values of the features of the feature vector define coordinates for the feature vector, such that each feature vector can be mapped to a feature space. In the example technique shown in FIG. 11, processor 40 determines whether the sequential feature vectors (e.g., a progression of coordinate points in the feature space) are approaching the classification boundary (177). In some examples, processor 40 determines the features vector based on a segment of the patient parameter signal, where the segment has a predetermined duration. Each feature vector can be determined based on a different portion of the segment of the patient parameter signal. In this way, the trajectory of feature vectors within the feature space may indicate the progression of the patient condition for a predetermined duration of time. In other examples, processor 40 continuously determines feature vectors based on the patient parameter signal. In this example, processor 40 monitors the trajectory of the feature vectors over an unknown, unspecified period of time. However, processor 40 can evaluate a path of a trajectory based on a limited (e.g., predetermined) number of feature vectors for, e.g., ease of processing. For example, processor 40 can evaluate the patient state based on a trajectory of about 2 to about 100 feature vectors, such as about 2 to about 4 feature vectors. The predetermined number of feature vectors can be based on the most recent segment of the patient parameter signal. In this way, processor 40 can evaluate the patient state based on a segment of the patient parameter signal that is relevant to the current patient state.

Regardless of the duration of time for which the trajectory is observed or the number of feature vectors in the trajectory, the location of the sequential feature vectors within feature space 128 (FIG. 10) may indicate whether the patient state is changing, which may indicate a prospective patient state change. For example, the feature vectors over time may define a trajectory toward the classification boundary, thereby indicating patient 14 may be on the course of an imminent or probable patient state change. In this way, the trajectory of feature vectors determined based on sequential segments of a sensed patient parameter signal can be used to predict an occurrence of a patient state.

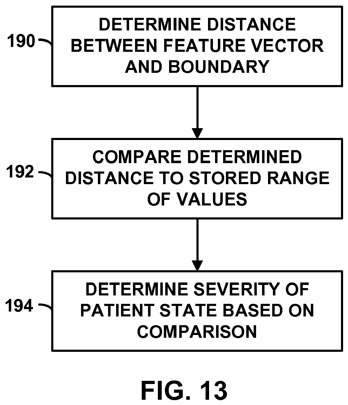

In some examples, processor 40 determines whether the feature vectors over time define a trajectory toward the classification boundary (177) by determining a distance between the feature vectors and the classification boundary, e.g., as described with respect to FIGS. 13-14B. If the distance between the feature vectors for consecutive segments of the patient parameter signal (which may not necessarily be continuous segments) and the classification boundary decrease over time, processor 40 may determine that the feature vectors are defining a trajectory toward the classification boundary. The distance can be the absolute magnitude of a perpendicular line extending between the feature vector in the feature space and the classification boundary. The trajectory can be, but need not be linear. In some examples, processor 40 determines that the feature vectors are defining a trajectory toward the classification boundary if each subsequent feature vector (e.g., the feature vectors determined based on subsequent segments of a patient parameter signal) in the trajectory is closer to the classification boundary than the previous feature vector.

In other examples, each subsequent feature vector in the trajectory need not necessarily be closer to the classification boundary than the previous feature vector, but the direction of the trajectory can be defined by nonsequential feature vectors. For example, a trajectory towards the classification boundary can include a first feature vector that is a first distance from the classification boundary and determined at a first time, a second feature vector that is a second distance from the classification boundary and determined at a second time following the first time, a third feature vector that is a third distance from the classification boundary and determined at a third time following the second time, and a fourth feature vector that is a fourth distance from the classification boundary and determined at a fourth time following the third time.

In some examples, processor 40 determines that the feature vectors are defining a trajectory toward the classification boundary over time when the fourth feature vector is closer to the classification boundary than the third feature vector, the third feature vector is closer to the classification boundary than the second feature vector, and the second feature vector is closer to the classification boundary than the first feature vector. In other examples, processor 40 determines that the feature vectors are defining a trajectory toward the classification boundary over time when the fourth feature vector is closer to the classification boundary than any one or more of the first, second or third feature vectors (even if, e.g., the second or third feature vectors are further from the classification boundary than the first feature vector), if the third feature vector is closer to the classification boundary than any one or more of the first or second feature vectors, or if the second feature vector is closer to the classification boundary than the first feature vector.

If processor 40 determines that the feature vectors determined based on the patient parameter signal are not defining a trajectory toward the classification boundary over time, processor 40 may continue monitoring the patient parameter signal (160) and the trajectory of feature vectors over time.

On the other hand, if processor 40 determines that the feature vectors determined based on the patient parameter signal are defining a trajectory toward the classification boundary over time, processor 40 generates a prospective patient state indication (178) that indicates the patient state associated with the other side of the classification boundary, to which the trajectory of feature vectors is approaching over time, is imminent or at least likely to occur. The prospective patient state indication can be, for example, a value, flag or signal that is stored in memory 42 of IMD 16 or another device (e.g., programmer 28). In examples in which the trajectory of the feature values does not cross the classification boundary, the generation of the prospective patient state indication does not signify that processor 40 detected the actual occurrence of the patient state, but, rather, that processor 40 predicted the occurrence of the patient state based on the trajectory of the feature values.

In the example shown in FIG. 11, upon generating the prospective patient state indication, processor 40 can initiate the proper course of action (e.g., deactivating, initiating or adjusting therapy delivery, generating a patient notification or initiating, deactivating or adjusting the recording of the patient parameter signal). In some examples, processor 40 initiates the proper course of action (e.g., initiating therapy delivery or generating a patient notification) when the distance between a feature vector and the classification boundary is less than or equal to a predetermined threshold, which may be stored in memory 42. In other examples, processor 40 initiates the proper course of action (e.g., initiating therapy delivery or generating a patient notification) when a threshold number of feature vectors for consecutive segments of the patient parameter signal define a trajectory toward the classification boundary. The threshold number of feature vectors in the trajectory that are used to determine a trajectory is moving towards a classification boundary can be predetermined by a clinician and stored by memory 42 (FIG. 2) of IMD 16, memory 62 (FIG. 3) of programmer 28 or a memory of another device.

Initiating the course of action prior to the patient 14 reaching the patient state may help prevent the occurrence of the patient state or at least mitigate the severity of any symptoms associated with the patient state. The trajectory toward the classification boundary that is defined by the feature vectors may indicate that it is likely patient 14 will eventually reach the patient state. Thus, any prophylactic therapy delivery may be useful for managing the patient condition. In addition, providing therapy prior to patient 14 actually achieving the patient state may be more useful in some examples than providing therapy after patient 14 is actually in the patient state. For example, if the patient state is a seizure disorder, providing therapy delivery prior to the seizure state may be more useful for preventing or mitigating the seizure than delivering therapy after patient 14 is in the seizure state. Similarly, generating a patient notification prior to the seizure may be more useful for providing patient 14 with notice about the occurrence of the seizure than delivering the notification after patient 14 is in the seizure state. For example, the notification prior to the occurrence of the seizure state may provide patient 14 with adequate notice to a safe position prior to the onset of any debilitating effects of the seizure or otherwise prepare for the onset of the seizure (e.g., by stopping a vehicle if patient 14 is driving the vehicle).

As another example, if the patient state is a state in which one or more symptoms of a movement disorder are present, providing therapy delivery prior to the movement state may be more useful for helping patient 14 initiate and/or maintain movement than providing patient 14 with therapy after the movement disorder symptoms have presented. Delivery of therapy prior to the occurrence of one or more symptoms of a movement disorder may help minimize the perception of any movement disorder symptoms by patient 14. Predicting the occurrence of the movement disorder symptoms based on a trajectory of the feature vectors towards a classification boundary may help time the delivery of therapy such that patient 14 does not substantially perceive an inability to initiate movement or another effect of a movement disorder. This also applies to other patient states. In general, predicting the occurrence of the patient state based on a trajectory of the feature vectors towards a classification boundary delineating the patient state from another state may help time the delivery of therapy such that patient 14 does not substantially perceive symptoms associated with the patient state.

In some examples, it can also be useful to control stimulation generator 44 (or another therapy module) to adjust therapy delivery to patient 14 to a therapy setting that provides efficacious therapy to patient 14 during the posture state prior to the patient 14 occupying the patient state. For example, if the patient 14 feels more pain in a particular patient state, it can be useful to initiate therapy delivery for the particular posture state prior to patient 14 occupying the posture state such that there is no delay in the therapeutic benefits.

In some examples, depending on the patient and the type of patient parameter signal, the progression of the patient condition over time may provide a better indication of patient state compared to, for example, a discrete feature vector determined based on a single portion of a sensed patient parameter signal. For example, a discrete feature vector may be an outlier (e.g., based on a transient change in the patient parameter signal) and may not provide an accurate representation of the current patient state. On the other hand, the trajectory of feature vectors over time is based on a longer time window, and may provide a more robust and meaningful indication of the current patient state. In the case of patient posture states, the discrete feature vector may represent a transient posture state (e.g., an intermediary posture state occupied by patient during a transition between first and second posture states). On the other hand, a trajectory of feature vectors determined based on consecutive segments of a patient parameter signal indicative of patient posture or activity can indicate the change in the patient posture state over a longer range of time, and, therefore, may not consider patient 14 to be in a transient posture state, but, rather, approaching the second posture state. Therefore, therapy delivery to patient 14 can be controlled based on the detection of the second posture state.

In some examples, processor 40 (or a processor of another device, such as programmer 28) can determine an evaluation metric based on the trajectory of the feature vectors relative to the classification boundary defined by the SVM. The evaluation metric can be stored in memory 42 of IMD 16 or a memory of a device. A log of the evaluation metrics generated by processor 40 over time can provide data with which a clinician can evaluate the progression of the patient's condition, monitor the severity of the patient condition, and the like. The evaluation metric can indicate, for example, whether the patient's condition is improving (e.g., if the trajectory is approaching the classification boundary in examples in which patient 14 is currently in a negative patient state) or whether the patient's condition is worsening (e.g., if the trajectory is approaching the classification boundary in examples in which patient 14 is currently in a positive patient state). In addition, in some examples, the evaluation metric can indicate whether the patient is approaching a patient state transition (e.g., if the trajectory is approaching the classification boundary).

In some examples, the evaluation metric is a distance between at least one of the feature vectors of the trajectory and the classification boundary. The distance can be determined using any suitable technique, such as the techniques described below with respect to FIG. 13. In some examples, the evaluation metric is a mean or median distance determined based on the distances of two or more feature vectors in the trajectory to the classification boundary. In other examples, the evaluation metric is a smallest distance between any one of the feature vectors in the trajectory and the classification boundary. In yet other examples, the evaluation metric is a distance between the feature vector determined based on the most recent segment of the patient parameter signal (e.g., the segment of the patient parameter signal that was observed at the latest point in time) and the classification boundary. In these examples, the evaluation metric can indicate whether patient 14 is approaching a patient state change.

In some cases, a relatively small (e.g., compared to a predetermined threshold value) distance between at least one of the feature vectors of the trajectory and the classification boundary can indicate that the patient's condition is improving. For example, if patient 14 is in a negative patient state and the distance between one or more feature vectors and the classification boundary is decreasing, the distance can indicate that the patient is approaching a more positive patient state (e.g., a non-seizure state or a positive mood state in which one or more symptoms of the patient's mood disorder are not present). However, in some examples, a relatively small distance can indicate that the patient's condition is worsening. For example, if patient 14 is in a positive patient state and the distance between at least one of the feature vectors of the trajectory and the classification boundary decreases or is less than a predetermined threshold value, the trajectory may indicate that patient 14 is approaching a more negative patient state (e.g., a seizure state or a more severe seizure state, or a negative mood state, such as a depressive or anxious mood state). In addition, in some examples, a plurality of evaluation metrics can indicate whether the patient is approaching a patient state transition (e.g., if the trend in distances between the feature vectors and classification boundary is decreasing, the trajectory is approaching the classification boundary).

In the example in which the evaluation metric is based on a distance between the feature vector determined based on the most recent segment of the patient parameter signal and the classification boundary, the evaluation metric may indicate, based on the magnitude of the distance to the classification boundary, whether patient 14 is close to transitioning to a different patient state. A relatively small magnitude of the distance of the feature vector to the classification boundary may indicate that patient 14 is approaching a transition to a different patient state or that the patient state transition is imminent. The clinician can determine the metric (e.g., distance value) that indicates that the patient state transition is imminent. In some cases, this metric can be determined during the SVM training stage, while in other cases, the metric can be determined following a monitoring period in which patient states are detected using the SVM-based classification algorithms described herein and patient state indications are stored in memory for later evaluation.

As described above, the trajectory can have a known (e.g., predetermined or calculated) number of feature vectors. In these examples, in addition to or instead of a distance between one or more feature vectors of the trajectory and the classification boundary, the evaluation metric can include the number of feature vectors or a percentage of the feature vectors within the trajectory that are less than a threshold distance away from the classification boundary. The threshold can be predetermined, e.g., by a clinician or the supervised machine learning technique, and stored in memory 42 of IMD 16 or a memory of another device.

In addition, in some examples, the evaluation metric can include the number of consecutive feature vectors (e.g., determine based on a continuous segment of the patient parameter signal) of a trajectory that are approaching the classification boundary.

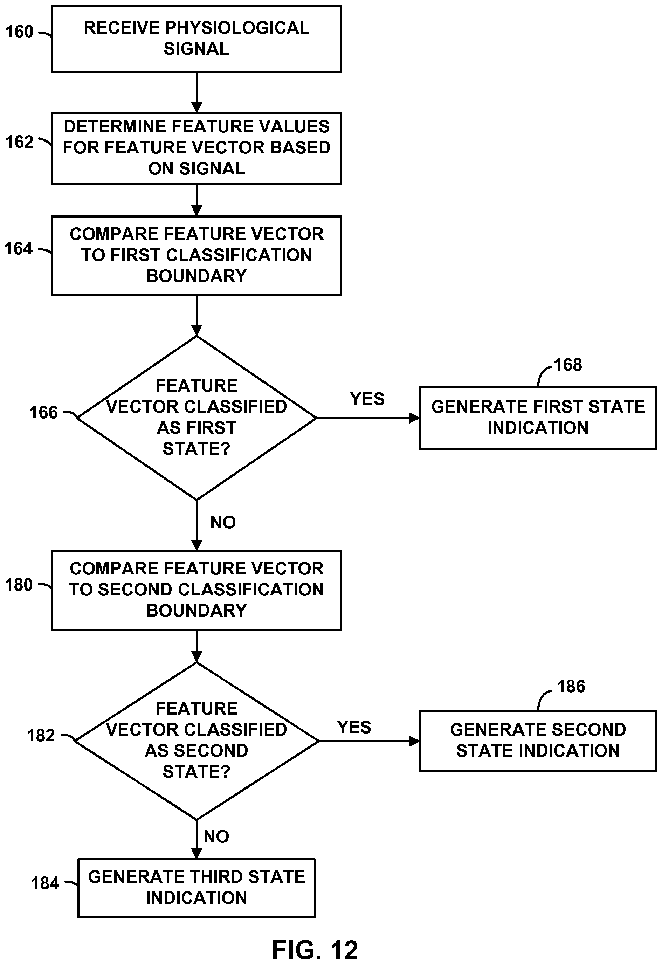

FIG. 12 is a flow diagram of an example technique processor 40 may implement to determine which of three patient states are indicated by a patient parameter signal. As with the technique shown in FIG. 9, in the technique shown in FIG. 12, processor 40 receives a signal indicative of a patient parameter from motion sensor 36 (FIG. 2), sensor 38 (FIG. 1) or sensing module 46 (FIG. 2) or another sensing module (160) and determines values for a feature vector based on a portion of the sensed signal (162). Processor 40 compares the determined feature vector to a first classification boundary determined by a first SVM algorithm (164) to determine whether patient 14 is in a first state or is not in the first state. The boundary may be linear (e.g., linear boundary 130 in FIG. 5) or nonlinear (e.g., nonlinear boundary 140 in FIG. 7). Processor 40 maps the determined feature vector to the feature space and determines the side of the boundary in which the feature vector lies.

If the feature vector lies on a side of the boundary associated with the first patient state, processor 40 classifies the determined feature vector in the feature space associated with the first state and determines that patient 14 is in the first state. Processor 40 may then generate a first state indication (168). On the other hand, if the feature vector does not lie within a side of the classification boundary associated with the first patient state, processor 40 determines that patient 14 is not in the first state.

In order to further classify the patient state, processor 40 implements additional classification boundaries. The classification boundaries can be generated by an SVM based on the same or different training data. In the example shown in FIG. 12, in order to determine whether the determined feature vector indicates a second or a third patient state, processor 40 implements a classification boundary generated by the first SVM algorithm or a second SVM algorithm and compares the determined feature vector to the second classification boundary (180). Processor 40 determines whether the feature vector indicates patient 14 is in the second state (182). In particular, if the feature vector lies within a side of the second classification boundary associated with a second patient state, processor 40 classifies the determined feature vector in the feature space associated with the second state and determines that patient 14 is in the second state. Processor 40 may generate a second state indication (182). As with the first state indication, the second state indication may be, for example, a value, flag or signal that is stored in memory 42 of IMD 16 or another device (e.g., programmer 28). In some examples, processor 40 determines whether a predetermined number (e.g., four) of consecutive points are on one side of the boundary before determining patient 14 has changed states to the second state. If the second SVM algorithm indicates that patient 14 is not in the second state (182), processor 40 determines that patient 14 is in a third state and generates a third state indication (184).

In the examples described herein, each SVM algorithm provides a binary indication of whether patient 14 is in a particular patient state. In examples in which classification of more than two states is desirable, processor 40 may use any suitable number of SVM algorithms to determine whether patient 14 is in one of a plurality of patient states. Processor 40 may compare a feature vector determined based on a sensed patient parameter to any number of classification boundaries of respective SVM-based classification algorithms. Each SVM-based classification algorithm may be used to further differentiate a patient state. Processor 40 may make the comparison in parallel or in series.

In some examples, classification of more than two patient states is desirable when the patient states are different posture states. For example, with respect to the technique described with respect to FIG. 12, the first state may be a lying down state, the second state can be an upright and active state, and the third state can be an upright state. As another example, the first state may be a lying front posture state, the second state can be a lying right posture state, and the third state can be lying left posture state. Any possible number and order of posture state detections can be implemented using the one or more SVM-based algorithms.

In addition, in some examples, classification of more than two patient states can be useful for characterizing a severity of a particular patient state in which one or more symptoms of a patient episode or event are present (e.g., a seizure episode, a movement disorder episode or a mood state disorder episode). For example, an electrographic seizure associated with a motor component (e.g., a tonic clonic seizure) can be considered relatively severe compared with sensory seizure (e.g., an electrographic seizure not associated with a motor component). With respect to the technique described with respect to FIG. 12, the first state may be a non-seizure state, the second state can be a sensory seizure state, and the third state can be a motor seizure state. Any possible number and order of seizure state detections can be implemented using the one or more SVM-based algorithms. Other types of severity classifications for seizure states as well as other patient disorders (e.g., mood state disorders) are also contemplated. Different classification boundaries that distinguish between the patient states of varying severity can be determined based on training data associated with patient states having different levels of severity. By implementing the multiple classification boundaries that define a feature space into different sections that are associated with different levels of severity of a particular patient event, the technique shown in FIG. 12 can be useful for determining the severity of a particular patient state.

In some examples, depending on the patient state, processor 40 or a processor of another device (e.g., programmer 28) determines a severity of the patient state based on a common classification boundary generated by a SVM algorithm. For example, the severity of a seizure state, a depressive mood state, an anxious mood state, a manic mood state, and the like may be determined by determining a distance between the feature vector on which the patient state classification was made and the classification boundary of the SVM algorithm.

FIG. 13 is a flow diagram illustrating an example technique with which processor 40 may determine an evaluation metric (e.g., a severity metric) with the aid of a classification boundary generated by a SVM algorithm. The evaluation metric may be a value or any other indication that can be used to evaluate a detected patient state, and, in some cases, compare a plurality of detected patient states with each other. The evaluation metrics can be stored in a memory of a device, such as IMD 16 or programmer 28 for later analysis by a clinician. However, the evaluation metrics can also be generated as needed by the clinician based on stored patient parameter signals. After determining patient 14 is in a particular patient state and mapping a determined feature vector to a predetermined feature space, processor 40 determines a distance between a determined feature vector and a classification boundary defined by a SVM algorithm (190). Example techniques for determining a feature vector based on a sensed patient parameter signal are described in further detail with reference to FIGS. 9 and 12, and example techniques of determining a feature space is described with reference to FIG. 4.

Processor 40 can determine the distance between a feature vector, e.g., determined based on a segment of a sensed patient parameter signal that indicates the current patient state, and a classification boundary defined by a SVM algorithm using any suitable technique. In some examples, processor 40 updates either Equation 1 or 2, which can also be used to determine the classification boundary, with the determined feature vector. The update to Equation 1 or 2 with the feature vectors results in a specific number, which correlates to the distance between the feature vector and the classification boundary. Processor 40 can determine whether the resulting value is positive or negative. A positive value can indicate that the feature vector is on a first side of the classification boundary and a negative value can indicate that the feature vector is on a second side of the classification boundary. In addition, the magnitude of the value determined based on Equation 1 indicates the distance between the feature vector and the classification boundary. In general, the value increases as the feature vector becomes further from the classification boundary, such that a relative small value indicates the feature vector is close to the classification boundary and a relatively large value indicates the feature vector is relatively far from the classification boundary.

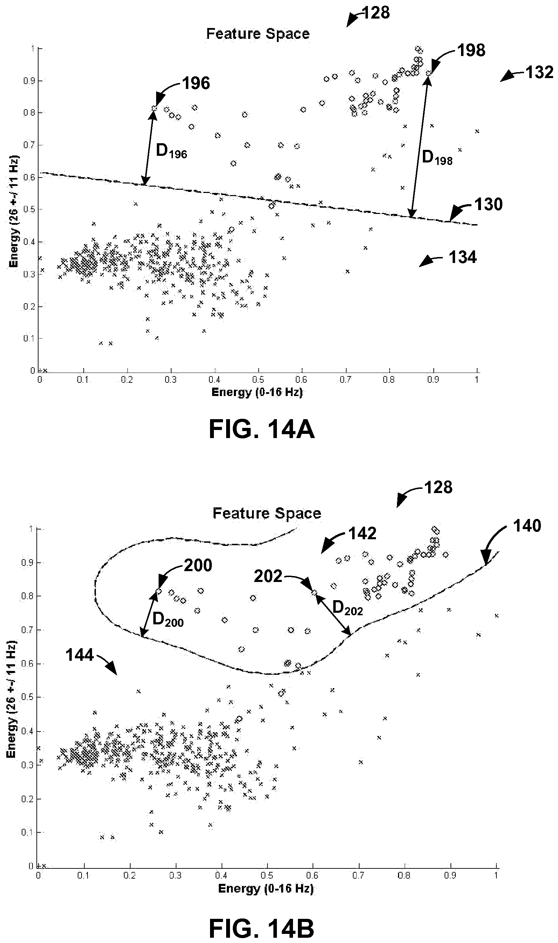

FIGS. 14A and 14B are conceptual illustrations of a feature space and illustrate how a distance between a classification boundary and a determined feature vector may be determined. In FIG. 14A, processor 40 determines feature vectors 196, 198 based on different portions of a sensed patient parameter signal and classifies feature vectors 196, 198 in region 132, which indicates patient 14 is in a first state (e.g., a seizure state). Feature vectors 196, 198 may be determined at different times, such that feature vectors 196, 198 provide a patient state indication for different periods of time. Feature vectors 196, 198 have different feature values. Processor 40 maps feature vectors 196, 198 to feature space 128 and determines a distance between each of feature vectors 196, 198 and linear boundary 130. In particular, processor 40 determines that feature vector 196 is a distance $D_{196}$ from linear boundary 130, where distance $D_{196}$ is measured in a direction substantially perpendicular to linear boundary 130. In addition, processor 40 determines that feature vector 198 is a distance $D_{198}$ from linear boundary 130, where distance $D_{198}$ is measured in a direction substantially perpendicular to linear boundary 130. As discussed above, in some examples, distances $D_{196}$ can be the value resulting from updating Equation 1 with feature vector 196, and distance $D_{198}$ can be the value resulting from updating Equation 2 with feature vector 198.

In FIG. 14B, which illustrates a feature space in which a nonlinear boundary 140 delineates first and second patient states, processor 40 determines feature vectors 200, 202 based on different portions of a sensed patient parameter signal at different times and classifies feature vectors 200, 202 in region 142, which indicates patient 14 is in a first state (e.g., a seizure state). Processor 40 maps feature vectors 200, 202 to feature space 128 and determines a distance between each of feature vectors 200, 202 and nonlinear boundary 140. In particular, processor 40 determines that feature vector 200 is a distance $D_{200}$ from nonlinear boundary 140, where distance $D_{200}$ is measured in a direction substantially perpendicular to nonlinear boundary 140. In addition, processor 40 determines that feature vector 202 is a distance $D_{202}$ from nonlinear boundary 140, where distance $D_{202}$ is measured in a direction substantially perpendicular to nonlinear boundary 140. As discussed above, in some examples, distances $D_{200}$ can be the value resulting from updating Equation 2 with feature vector 200, and distance $D_{202}$ can be the value resulting from updating Equation 2 with feature vector 202.

Returning now to the technique shown in FIG. 13, for each feature vector, processor 40 compares the determined distance between the determined feature vector and the classification boundary to each of a plurality of stored distance values (192). The distance values may be predetermined, e.g., by a clinician, and stored in memory 42 of IMD 16 or a memory of another device. Each stored value, which may be a range of values, may be associated with a particular severity metric. For example, the stored values may indicate that the further a feature vector is from a classification boundary, as indicated by the determined distance, the more severe the patient state. This may be because the classification boundary delineates first and second patient states, and, thus, the further a feature vector lies from the classification boundary, the further the feature vector lies from the other patient state. For example, a second patient state may indicate that patient 14 is not in a first state. Thus, the second state may be a relatively lowest severity rating for the first state because of the nonexistence of the first state.

A plurality of distance values is stored in order to differentiate between levels of the patient state, where the different levels can be associated with, for example, different patient symptoms, different degrees of the patient symptom or different perceptions of the patient state by the patient. In this way, the distance values represent different severity metrics. A severity metric may indicate the relative severity of one or more symptoms of the patient state. For example, in the case of a seizure state, the severity metric may indicate whether the seizure was associated with a motor component (e.g., a tonic clonic seizure). As another example, in the case of a depressive state, the severity metric may indicate the severity of one or more symptoms of the depression (e.g., anhedonia). Any suitable number of severity metrics may be used. Processor 40 determines the severity of the patient state based on the comparison of the determined distance between the determined feature vector and the classification boundary to the stored values (194).

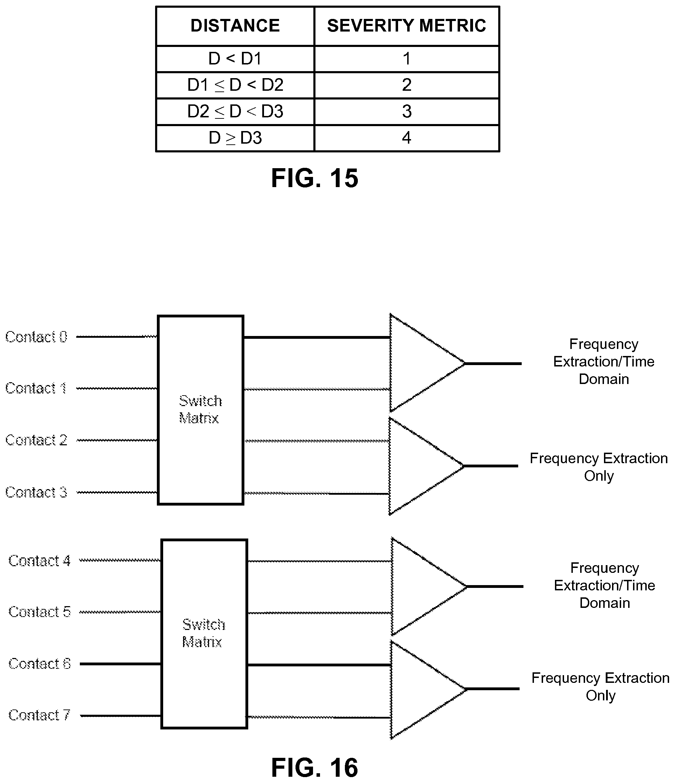

An example of a data structure that associates each of a plurality of distance ranges of a severity metric is shown in FIG. 15. The data structure may be stored in memory 42 of IMD 16 (FIG. 2), memory 62 of programmer 28 (FIG. 3) or a memory of another device. The data structure includes a column that lists a plurality of distance ranges and a column that indicates a severity metric associated with a respective distance range. In the example shown in FIG. 15, the data structure indicates that if a determined distance D (between a determined feature vector and a classification boundary of a SVM algorithm) is less than a predetermined distance D1, the severity metric is "1," where the severity metric indicates the severity of the patient state. In addition, the data structure indicates that if determined distance D is greater than or equal to distance D1, but less than distance D2, the severity metric for the patient state indicated by the associated feature vector is "2." The data structure also indicates that if determined distance D is greater than or equal to distance D2, but less than distance D3, the severity metric for the patient state indicated by the associated feature vector is "3." Finally, the data structure indicates that if the determined distance D is greater than or equal to distance D3, the severity metric is "4."

Distances D1, D2, and D3 can be determined using any suitable technique. In some examples, processor 60 of programmer 28 or a processor of another device (e.g., IMD 16) automatically determines distances D1, D2, and D3 based on patient input during the patient state classification algorithm training stage. For example, if patient 14 provides input indicating the occurrence of a patient event (e.g., a seizure, a movement state, a particular patient posture, a particular mood state or a compulsion), patient 14 can provide feedback regarding the severity of the patient event. Processor 60 can organize the training feature vectors into different severity categories based on the patient feedback and determine the distance ranges for each of the severity categories based on the distances of the training feature vectors to the classification boundaries. In other examples, distances D1, D2, and D3 can be determined by a clinician, alone or with the aid of programmer 28. Regardless of how the distances are determined, the distances can be determined based on training data specific to patient 14 or data for more than one patient.

Patient 14 or another user can provide feedback regarding the severity of a particular patient event (or patient state) using any suitable mechanisms. In some examples, a numeric rating scale can be used. In other examples, such as in examples in which IMD 16 is used to deliver therapy for pain management, the Wong-Baker FACES Pain Rating Scale or the McGill Pain Questionnaire can be used. In examples in which the patient event is mood state, the Beck Depression Inventory, Hamilton Rating Scale for Depression (HAM-D) or the Montgomery-Asberg Depression Rating Scale (MADRS) can be used to assess the severity of the patient state. The Beck Depression Inventory and the HAM-D are both 21-question multiple choice surveys that is filled out by patient 14, and the MADRS is a ten-item questionnaire. The answers to the questions may indicate the severity of patient symptoms or the general patient mood state, and processor 60 (or a clinician) may assign a severity rating to the indicated patient state based on the subjective patient or patient caretaker evaluation.

Example systems and techniques for acquiring patient data (e.g., patient parameter signal and/or subjective patient feedback regarding the severity of a patient event) regarding a patient event are described in commonly-assigned U.S. Patent Application No. 12/236,211 by Kovach et al., entitled, "PATIENT EVENT INFORMATION," which was filed on September 23, 2008. As described in U.S. Patent Application Serial No. 12/236,211 by Kovach et al., processor 60 of programmer 28 or another computing device may generate an event marker upon activation of an event indication button of programmer 28 by patient 14. For example, if patient 14 detects a patient event, patient 14 may activate the event indication button, and, in response, processor 60 may generate an event marker. The patient may provide event information relating to the patient event. For example, the event information may include the type of patient event, the patient's rating of the severity of the patient event, the duration of the patient event, and the like. The segment of the patient parameter signal corresponding in time to the event indication can then be used to determine a feature vector, and a distance between that feature vector and a classification boundary determined using any suitable supervised machine learning technique can be used to generate the distance ranges used to provide severity metrics.

The severity metrics 1-4 may be a part of a graduated scale, whereby a severity metric of "4" that is associated with a feature vector indicates that the patient state associate with the feature vector was a more severe patient state (e.g., a more severe seizure or a patient mood state) than a patient state associated with a severity metric of "1." Other types of severity metrics are contemplated and need not be on a graduated scale. For example, the severity metrics may be binary and indicate whether a detected patient state was severe or not severe. The table shown in FIG. 15 is for purposes of example only. In other examples, any suitable number of distance ranges and associated severity metrics may be defined, and the data structure may have a structure other than a table.

Processor 40 may reference the data structure shown in FIG. 15 to determine the relative severity of the patient states indicated by the determined feature vectors 196, 198 (FIG. 14A). For example, processor 40 may compare distance $D_{196}$ between feature vector 196 and linear boundary 130 (FIG. 14A) to the plurality of stored distance ranges stored by the data structure shown in FIG. 15. In the example shown in FIG. 14A, processor 40 determines that determined distance $D_{196}$ is greater than D1, but less than D2, and, thus, processor 40 associates the patient state detected at the time associated with feature vector 196 with a severity metric of "2." The detected patient state and associated severity metric may be stored in memory 42 of IMD 16 (FIG. 2), memory 62 of programmer 28 (FIG. 3) or a memory of another device.

Processor 40 may also compare distance $D_{198}$ between feature vector 198 and linear boundary 130 (FIG. 14A) to the plurality of stored distance ranges stored by the data structure shown in FIG. 15. In the example shown in FIG. 14A, processor 40 determines that determined distance $D_{198}$ is greater than D3. Thus, processor 40 may associate the patient state detected at the time associated with feature vector 198 with a severity metric of "4." Because the distance $D_{198}$ between feature vector 198 and linear boundary 130 is greater than distance $D_{196}$ between feature vector 196 and boundary 130, processor 40 determines that the patient state detected at the time associated with feature vector 198 is more severe than the patient state detected at the time associated with feature vector 196. This difference in severity is indicated by the different severity metrics associated with the respective feature vectors.

Processor 40 may also reference the data structure shown in FIG. 15 to determine the relative severity of the patient states determined based on feature vectors 200, 202 (FIG. 14B) that are mapped to feature space 128 with a nonlinear boundary 140. In some examples, depending upon the distance ranges stored by the data structure shown in FIG. 15, processor 40 may determine that because distance $D_{202}$ between feature vector 202 and boundary 140 is greater than distance $D_{200}$ between feature vector 200 and boundary 140. As a result, processor 40 may determine that the patient state detected at the time associated with feature vector 202 is more severe than the patient state detected at the time associated with feature vector 200. In other examples, depending upon the distance ranges stored by the data structure shown in FIG. 15, processor 40 may determine that although distance $D_{202}$ is greater than distance $D_{200}$, the patient states detected at the times associated with feature vectors 200, 202 are associated with the same severity metric, thereby indicating the same relative severity compared to other detected patient states.

In each of these examples, distances $D_{196}$, $D_{198}$, $D_{200}$, and $D_{202}$ may be normalized such that comparison to each other may be useful. In addition, in other examples, processor 60 of programmer 28 may determine the severity metric for each detected patient state.

Processor 40 of IMD 16, processor 60 of programmer 28 or a processor of another device may track the severity of the patient's states (and, in some cases, the progression of the patient condition) by determining a maximum distance that a feature vector on one or both sides of a classification boundary achieves during a period of time or tracking a trend in the distances of determined feature vectors over time. Either the maximum distance over time or the determined distance over time may indicate, for example, whether the patient's condition is improving or worsening. For example, if feature vector 196 is determined at a first time, processor 40 may store distance $D_{196}$ (or the severity metric associated with feature vector 196 and determined based on distance $D_{196}$) as a baseline state of patient 14 or a current state of patient 14. Processor 40 may detect feature vector 198 at a subsequent time and determine that $D_{198}$, which indicates the relative severity of the patient state at the time associated with feature vector 198. If processor 40 determines that distance $D_{198}$ is greater than distance $D_{196}$, thereby indicating the severity of the most recently detected state has increased, processor 40 may determine that the patient's condition is worsening.

In addition to a severity metric, other types of metrics may be determined based on a determined feature space and feature vectors, which are each indicative of a patient state detection. For example, processor 40 (or processor 60 of programmer 28 or a processor of another device) may track the duration that patient 14 occupied a particular patient state by determining the number of feature vectors mapped to the side of the boundary of the feature space 128 associated with the patient state. In some examples, processor 40 determines a feature vector based on a predetermined patient parameter signal duration. The duration may be, for example, about one second to one minute or more (e.g., on the order of hours). Thus, each feature vector may indicate the state that patient 14 occupied for the predetermined duration of time.

The feature vectors on a first side of the classification boundary defined by a SVM algorithm may be totaled and multiplied by the predetermined duration of time to determine the duration of time that patient 14 occupied the first patient state associated with the first side of the classification boundary. The feature vectors on the second side may also be totaled and multiplied by the predetermined duration of time to determine the duration of time that patient 14 occupied the second patient state associated with the second side of the classification boundary.

As previously indicated, in some examples, processor 40 determines patient 14 has changed from state to another state only if multiple feature vectors determined based on sequential segments of a patient parameter signal indicate the state change. Thus, if one feature vector falls within a region associated with a patient state that is different than the previous state determination, processor 40 may continue monitoring the patient parameter signal and determining feature vectors based on consecutive segments of the patient parameter signal over time to determine whether additional feature vectors indicate the state change.

### SVM Example

An evaluation of various automated seizure detection algorithms was performed using stored ECoG signals of a patient with a seizure disorder. The SVM Example demonstrates that a SVM-based algorithm for detecting a seizure state resulted in improved sensitivity, specificity, latency and power consumption relative to other automated seizure detection techniques. This suggests that a SVM algorithm for detecting any patient state based on a sensed patient parameter signal may be useful and, in some cases, advantageous over existing patient state detection algorithms.

In the SVM Example, a sensing module that includes a chopper-stabilized superheterodyne instrumentation amplifier and a signal analysis unit that extracts a selected frequency band of a sensed ECoG signal to a baseband was used. The sensing module utilized a serial port for real-time data uplink of the stored ECoG signals. A SVM algorithm was trained using one set of stored ECoG signals and uploaded into a programmable integrated circuit (PIC) (R) processor (made available by Microchip Technology Inc. of Chandler, Arizona), which may be a part of the sensing module or separate from the sensing module. Because the sensing module was configured to extract the spectral energy features of the ECoG signal, the digitization of the ECoG signal was performed at a relatively slow rate of about 1 Hz.

Classification of the sensed ECoG signal as indicating a seizure state or a non-seizure was performed by the PIC processor based on another set of stored ECoG signals using three different algorithms. In a first algorithm (ALGORITHM 1), an ECoG signal was determined to indicate a seizure state if the normalized spectrum of a portion of the ECoG signal was greater than a threshold value, as described above with respect to the patient non-specific algorithm for triggering the recording of training data. Only one threshold was used for the first seizure detection algorithm, and the threshold was not specific to the patient, but was intended for use in a generic seizure detection algorithm for a plurality of patients. In a second algorithm (ALGORITHM 2), a single linear classification boundary defined by a SVM algorithm was used to classify portions of the ECoG signal as indicative of a seizure state or a non-seizure state. In a third algorithm (ALGORITHM 3), a nonlinear classification boundary defined by a SVM algorithm was used to classify portions of the ECoG signal as indicative of a seizure state or a non-seizure state. The linear and non-linear classification boundaries were determined based on training data that included approximately 81 hours of intracranial EEG (IEEG) collected from 17 adult subjects. On average, approximately 4.5 hours of recording time containing 3 seizures were available per patient. For each patient, a clinician identified the onset time of all seizures in order to identify the training data. At a later time, the two sensing channels that demonstrated the earliest signs of seizure activity for a specific patient were selected.

Due to the small number of seizures available for each patient, a leave-one-out testing methodology was adopted. For example, for a patient recording of IEEG data consisting of $K$ ten-minute blocks of IEEG data containing $L$ number of seizures. The patient-specific classification boundary was determined based on $K/2$ data blocks containing $L-1$ seizures. Next, the performance of both the patient-specific and patient non-specific detectors were assessed on the remaining K/2 blocks containing the $L^{th}$ seizure. This was repeated $L$ times so that the ability of each of the seizure detection algorithms was tested.

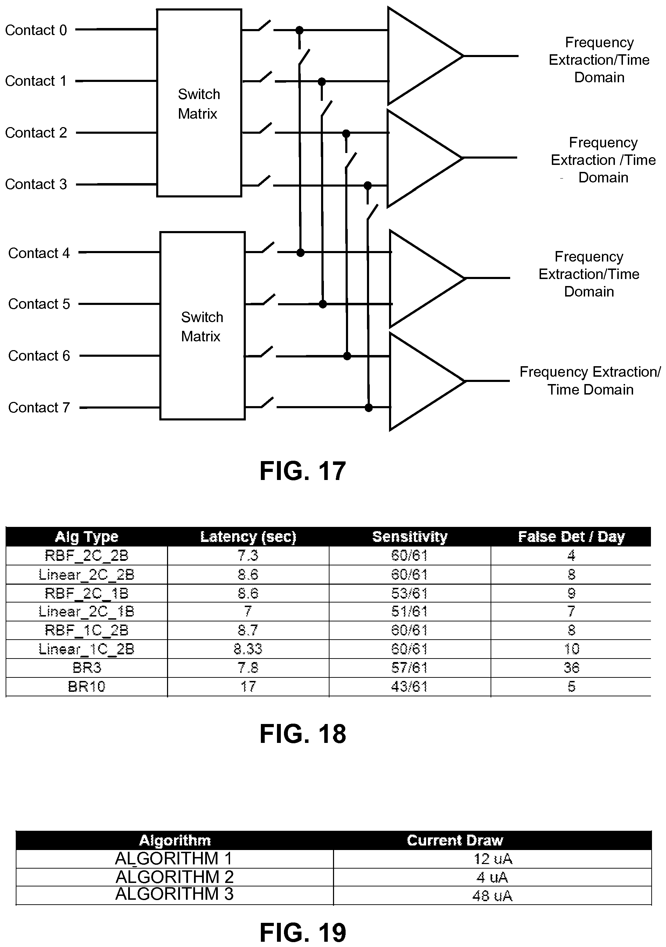

FIG. 16 is a conceptual block diagram of the sensing module circuitry that was used for the SVM Example. FIG. 17 is another conceptual block diagram of a sensing module circuitry that may be used in an IMD 16 to sense one or more physiological signals and extract specific frequency band components of the sensed signals. In FIG. 17, switches may be opened or closed to establish more combinations of "Contacts" compared to the circuit shown in FIG. 16. The "Contacts" may be, for example, electrodes of an implantable medical lead that is positioned to sense bioelectrical brain signals within a brain of a patient (e.g., electrodes 24, 26 shown in FIG. 1).

As FIGS. 16 and 17 show, different sensing channels ere used to extract either the frequency component (indicated as "Frequency Extraction") of an ECoG signal or to sense the time-domain ECoG signal. In the case of seizure detection, the time-domain signal may be important to SVM training because a clinician may determine which data segments of an ECoG signal (or other sensed signal) contains a seizure and which data segments do not based on the time-domain signal. With the sensing circuit architecture shown in FIG. 16, it may not be possible to gather more than one spectral feature vector simultaneously with time-domain data. Thus, it may be useful to enable a more robust SVM training with the architecture shown in FIG. 17 by having two sensing channels that extract a different frequency component of a sensed signal.

FIG. 18 is a table that compares different sensing capabilities based on the seizure detection latency, sensitivity, and the number of false detections per day for seizures detected using the signals generated by a conceptual sensing module including the respective sensing capability. Latency may be, for example, the duration of time between the onset of the seizure and the detection of the seizure by the PIC processor. A negative latency may indicate that the seizure was detected before the onset of the seizure, where the "onset" may be defined according to different criteria and may be specific to a particular clinician's criteria. A false detection was determined to be a seizure detection made during any window of time noted by a clinician to be free of seizure activity.

The labels used in FIG. 18 are as follows:

**RBF_2C_2B:** Nonlinear SVM (ALGORITHM 3) using two sensing channels and two frequency bands per channel
**Linear_2C_2B:** Linear SVM (ALGORITHM 2) using two sensing channels and two frequency bands per channel
**RBF_1C_2B:** Nonlinear SVM (ALGORITHM 3) using one sensing channel and two frequency bands
**Linear_1C_2B:** Linear SVM (ALGORITHM 2) using one sensing channel and two frequency bands
**RBF_2C_1B:** Nonlinear SVM (ALGORITHM 3) using two channels and one frequency band per channel
**Linear_2C_1B:** Linear SVM (ALGORITHM 2) using two channels and one frequency band per channel
**BR 3 Sec:** ALGORITHM 1 with a three second temporal threshold for determining the amplitude for comparing to the seizure detection threshold
**BR 10 Sec:** ALGORITHM 1 with a ten second temporal threshold for determining the amplitude for comparing to the seizure detection threshold

As the table shown in FIG. 18 indicates, the PIC processor exhibited the best latency, sensitivity, and the lowest number of false detections per day while implementing ALGORITHM 3 and using two sensing channels with two extracted frequency bands per channel. In situations in which sensing a physiological signal with two channels and two bands per channels is not feasible, e.g., because of sensing hardware limitations, the data shown in FIG. 18 suggests that a sensing architecture including one sensing channel with two frequency bands provides a relatively low latency with a relatively high sensitivity, while minimizing the number of false detections per day.

The table shown in FIG. 18 compares the performance of the different seizure detection algorithms implemented by the PIC processor. The table shown in FIG. 18 also indicates that seizure detection using ALGORITHM 2, which is a SVM algorithm using a linear classification boundary, results in a better latency, sensitivity, and lower number of false seizure state detections per day compared to the existing techniques (ALGORITHM 1) that rely on a single threshold amplitude value that is not specific to a patient to detect a seizure. In addition, the table shown in FIG. 18 also indicates that seizure detection using ALGORITHM 3, which is a SVM algorithm that uses a nonlinear classification boundary, results in a better sensitivity compared to ALGORITHM 1 with a comparable latency and number of false seizure state detections per day. The rate of false detections can be reduced by extending the duration constraint of ALGORITHM 1 to 10 seconds, but FIG. 18 suggests that extending the duration of a sampled bioelectrical brain signal comes at the price of added latency and reduced sensitivity.

FIG. 19 is a table that compares a current draw for the seizure detection algorithms that were implemented on using a prototype implantable device, which included the PIC processor. The data shown in FIG. 19 suggests that the SVM algorithm using the linear boundary (ALGORITHM 2) drew the least amount of current during the seizure detection process (4 microamps compared to 12 microamps for ALGORITHM 1 and 48 microamps for ALGORITHM 3). It is believed that if a SVM algorithm including multiple linear boundaries is used by the PIC processor to detect a seizure state of a patient, the current draw shown in FIG. 19 would be multiplied by the number of linear boundaries used for the seizure detection. The data shown in FIGS. 18 and 19 indicate that the linear SVM algorithm (ALGORITHM 2) provides the best overall performance compared to the amount of current it draws.

As previously indicated, a SVM algorithm for determining whether patient 14 is in a particular state, e.g., detecting the patient state, may be useful for various patient states. An example technique for training and running a SVM algorithm for seizure detection is as follows:

1. Select one bioelectrical brain signal sensing channel, e.g., a channel that provides the best relative seizure detection.

2. Configure a sensing device (e.g., IMD 16) to record time-domain data and two frequency bands of the bioelectrical brain signal and enable recording (e.g., loop recording) to capture these channels.

3. Instruct patient 14 (and/or patient caregiver) on the provision of patient input via a programmer 28 or another input device, such patient 14 (or a caregiver) provides input indicating the occurrence of a seizure via the input device. Patient 14 also provides input indicating when a seizure is not occurring such that the medical device captures non-seizure data.

4. Capture training data. In some examples, the clinician can enable a seizure detection algorithm by the sensing device that utilizes a single threshold value that is not specific to patient 14 to trigger loop recording upon the detection of a seizure (e.g., the patient-non-specific algorithm discussed above). The seizure detection algorithm could be biased toward sensitivity to minimize the number of seizure occurrences that are not detected. In addition to or instead of the threshold value triggering of data, the storing of the training data can be initiated based on a timer or patient input, as described above. The automatic capturing of seizure data could take place during an ambulatory period where patient 14 is sent home and is not at the clinic.

5. Upload data onto a computing device, e.g., programmer 28.

6. Classify data segments as seizure and non-seizure.

7. Run automated SVM generation software (or another supervised machine learning technique) on the classified and separated data segments to determine one or more classification boundaries.

8. Load the one or more classification boundaries onto IMD 16.

9. Enable the SVM-based seizure detection algorithm that uses the one or more classification boundaries generated by the SVM. The seizure detection based on the classification boundary is used for various purposes, such as seizure burden monitoring, closed-loop delivery of therapy, providing patient notifications, and the like.

Other techniques for training and running a SVM-based algorithm for seizure detection are contemplated.

An example technique for training and running a SVM-based algorithm for detection of different movement disorder states (e.g., a first state in which one or more symptoms of a movement disorder of patient 14 are present and a second state in which the symptoms are not present) is as follows:

1. While patient 14 is not on medication for movement disorder therapy (e.g., stimulation therapy is disabled and no pharmaceutical agents have recently been ingested), the clinician determines the best sense electrode combination for determining the different movement disorder states. This could be performed by IMD 16 via an automated routine.

2. Determine the frequency band(s) that differentiate between the different movement disorder states.

3. Tune a sensing module to the selected frequency band(s) and enable loop recording to capture a bioelectrical brain signal in the selected channels.

4. Capture data for the first movement disorder state. The clinician may ensure correlation of the data with the first state by observing patient 14 and confirming that the selected movement disorder symptoms are present.

5. Deliver therapy (medication and/or stimulation therapy) to transition patient 14 to the second movement disorder state in which the selected movement disorder symptoms are mitigated or not present.

6. Capture bioelectrical brain signal data for the second movement disorder state. The clinician may ensure correlation of the data with the second state by observing patient 14 and confirming that the selected movement disorder symptoms are not present or mitigated.

7. Upload data onto a computing device, e.g., programmer 28.

8. Classify data segments as indicative of first or second states.

9. Run automated SVM generation software (or another supervised machine learning technique) on the classified and separated data segments

10. Load the one or more classification boundaries onto IMD 16.

11. Enable SVM-based algorithm that uses the one or more classification boundaries. The SVM-based algorithm runs and performs detection of the different movement disorder states for various purposes, such as movement disorder monitoring, closed-loop delivery of therapy, providing patient notifications, and the like.

In one example technique for training and running a SVM algorithm for detection of a depressed mood state and a non-depressed mood state, the SVM algorithm is based on an example in which an indicator of depression is the balance of energy in an alpha frequency band (e.g., approximately 5 Hz to approximately 13 Hz) of bioelectrical brain signals sensed in the two hemispheres of the cortex of brain 12 of patient 14. Thus, a sensing device that includes two sensing channels with one frequency band each may be used to sense the bioelectrical brain signals for implementation of the SVM algorithm. An example technique for training and running a SVM algorithm for detection of a depressed mood state and a non-depressed mood state is as follows:

1. Select two bioelectrical brain signal sensing channels, one from each hemisphere.

2. Tune the sensed signal to the alpha frequency band.

3. Tune a sensing device to the selected frequency band(s) and enable loop recording to capture these channels.

4. Instruct patient 14 (and/or patient caregiver) on the provision of patient input via a programmer 28 or another input device, such that patient 14 (or a caregiver) provides input indicating the occurrence of a depressed mood state via the input device. Patient 14 also provides input indicating a non-depressed mood state, such that the medical device captures non-depressed mood state bioelectrical brain signal data.

5. Capture depressed state data using patient event triggers. Data collection for SVM training may be done in an ambulatory manner because it may not be possible to capture data for each of the mood states in the clinic. The mood states are often slowly changing states that may be difficult to trigger in the clinic.

6. Capture non-depressed state data using patient event triggers.

7. For patients that also experience manic states, capture manic state data (could be a fully ambulatory period where patient 14 is sent home).

8. Upload data

9. Classify data segments as indicative of the depressed and non-depressed states.

10. For patients that also experience manic states, classify data segments as indicative of the manic and non-manic states.

11. Run automated SVM generation software (or another supervised machine learning technique) on the classified and separated data segments to generate separate classification boundaries for detecting the depressed and non-depressed states and the manic and non-manic mood states may also be generated.

11. Load classification boundaries onto IMD 16.

12. Enable SVM-based patient detection algorithm(s) using the classification boundaries. The SVM-based algorithm runs and performs mood state detection for various purposes, such as monitoring of the mood disorder of the patient, closed-loop delivery of therapy, providing patient notifications, and the like. The SVM-based algorithm for detection of a manic mood state and a non-manic state may be used in conjunction with the SVM-based algorithm for detection of a depressed mood state and a non-depressed mood state.

In some cases, a SVM-based algorithm may be used to detect a patient posture state. Posture state detection may be useful in various situations, such as to program and implement posture-responsive therapy delivery. Posture-responsive stimulation may be implemented for pain therapy.

An example technique for training and running a SVM-based algorithm for detection of an upright patient posture state based on a signal generated by a three-axis accelerometer is as follows, e.g., after an IMD is implanted in patient 14:

1. Collection of motion sensor (e.g., accelerometer) data is enabled, e.g., after implantation of an accelerometer in patient 14. A three-axis accelerometer can be used to provide three channels of data, whereby each channel is associated with a different axis.

2. Patient 14 occupies various postures and activities and data is logged for each of the known postures and activities. In some cases, a posture state can include posture and an activity level (e.g., an upright posture state may be differentiated from an upright and activate posture state).

3. Upload data.

4. Classify data segments as indicative of the upright and not-upright posture states. The "not upright" posture state may be any one or more other posture states that are not the upright posture state. For example, the "not upright" posture state can include a lying down posture state.

5. Run automated SVM generation software (or another supervised machine learning technique) on the classified and separated data segments to generate one or more classification boundaries for detecting the upright and not-upright posture states.

6. Load the one or more classification boundaries onto IMD 16.

7. Enable SVM-based algorithms using the one or more classification boundaries. The SVM-based algorithm(s) runs and performs posture state detection for various purposes, such as providing closed-loop delivery of therapy, providing patient notifications, and the like.

One or more additional SVM-based algorithms may be implemented to further refine the posture state detection. For example, after determining patient 14 is in an upright posture state with one SVM-based algorithm, processor 40 of IMD 16 may implement another SVM-based algorithm using a different classification boundary (and in some cases, different patient parameter signal features) to determine whether patient 14 is active or inactive to further determine whether patient 14 is in an upright and active posture state. As another example, after determining patient 14 is not in an upright posture state with one SVM-based algorithm, processor 40 of IMD 16 may implement another SVM-based algorithm to determine whether patient 14 is in a lying down posture state. Additional SVM-based algorithms may be used to further refine the lying down posture state, e.g., to determine which side of the body patient 14 is lying on.

The techniques described in this disclosure, including those attributed to programmer 28, IMD 16, or various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, embodied in programmers, such as physician or patient programmers, stimulators, image processing devices or other devices. The term "processor" or "processing circuitry" may generally refer to any of the foregoing logic circuitry, alone or in combination with other logic circuitry, or any other equivalent circuitry.

Such hardware, software, firmware may be implemented within the same device or within separate devices to support the various operations and functions described in this disclosure. While the techniques described herein are primarily described as being performed by processor 40 of IMD 16 and/or processor 60 of programmer 28, any one or more parts of the techniques described herein may be implemented by a processor of one of IMD 16, programmer 28, or another computing device, alone or in combination with each other.

In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

When implemented in software, the functionality ascribed to the systems, devices and techniques described in this disclosure may be embodied as instructions on a computer-readable medium such as RAM, ROM, NVRAM, EEPROM, FLASH memory, magnetic data storage media, optical data storage media, or the like. The instructions may be executed to support one or more aspects of the functionality described in this disclosure.

**Claims**

**1.** A system comprising:

means configured to generate a signal indicative of a parameter of a patient;

means (40) configured to determine a feature vector based on the signal; and

means (40) configured to apply a support vector machine based algorithm to classify a patient state based on the feature vector, wherein the support vector machine based algorithm defines a classification **characterised by** means (40) configured to determine a distance between the feature vector and the classification boundary after classifying the patient state based on the feature vector; and

means (40) configured to determine an evaluation metric for the patient state based on the distance, and wherein the evaluation metric indicates a severity of the patient state.

2. The system of claim 1, wherein the feature vector comprises respective values for at least two characteristics of the signal, wherein the characteristics each comprise at least one of an amplitude value or a frequency domain characteristic of the signal.

3. The system of claim 1, wherein the patient state comprises at least one of a seizure state, a mood state, a movement state or a patient posture state.

4. The system of claim 1, wherein the parameter comprises a least one of patient motion or activity, heart rate, respiratory rate, electrodermal activity, thermal activity or muscle activity.

5. The system of claim 1, wherein the means configured to determine the evaluation metric comprises means configured to access a data structure that associates a plurality of evaluation metrics with respective distances to the classification boundary.

6. The system of claim 1, further comprising means (40) configured to control therapy delivery to the patient based on the evaluation metric.

7. The system of claim 6, wherein the means configured to control therapy delivery to the patient selects at least one therapy parameter value based on the evaluation metric, the system further comprising a therapy module that delivers therapy to the patient based on the selected at least one therapy parameter value.

8. The system of claim 6, wherein the evaluation metric comprises the distance, and wherein the means configured to control delivery of therapy to the patient based on the evaluation metric at least one of deactivates, activates or adjusts therapy delivery to the patient if the evaluation metric is less than or equal to a threshold value.

9. The system of claim 6, wherein the evaluation metric comprises the distance, and wherein the means configured to control

- delivery of therapy to the patient based on the evaluation metric at least one of deactivates, activates or adjusts therapy delivery to the patient if the evaluation metric is greater than or equal to a threshold value.

10. The system of claim 1, further comprising a memory (42) that stores the evaluation metric.

11. The system of claim 1, wherein at least one of the means configured to determine the feature vector based on the signal, the means configured to apply the support vector machine based algorithm to classify the patient state based on the feature vector, the means configured to determine the distance between the feature vector and the classification boundary, and the means configured to determine an evaluation metric for the patient state based on the distance comprises a processor.

12. The system of claim 1, further comprising an implantable medical device (16) that comprises the means configured to generate the signal indicative of the parameter of the patient.

**Patentansprüche**

1. System umfassend:

   ein Mittel, das konfiguriert ist, um ein auf einen Parameter eines Patienten hinweisendes Signal zu erzeugen;
   ein Mittel (40), das konfiguriert ist, um einen Merkmalsvektor basierend auf dem Signal zu bestimmen; und
   ein Mittel (40), das konfiguriert ist, um einen auf einer Stützvektormaschine basierenden Algorithmus anzuwenden, um einen Patientenzustand basierend auf dem Merkmalsvektor zu klassifizieren, wobei der auf der Stützvektormaschine basierende Algorithmus eine Klassifizierung definiert, **gekennzeichnet durch** ein Mittel (40), das konfiguriert ist, um einen Abstand zwischen dem Merkmalsvektor und der Klassifizierungsgrenze nach dem Klassifizieren des Patientenzustandes basierend auf dem Merkmalsvektor zu bestimmen; und
   ein Mittel (40), das konfiguriert ist, um eine Evaluierungsmetrik für den Patientenzustand basierend auf dem Abstand zu bestimmen und wobei die Evaluierungsmetrik auf eine Schwere des Patientenzustandes hinweist.

2. System nach Anspruch 1, wobei der Merkmalsvektor betreffende Werte für zumindest zwei Charakteristika des Signals umfasst, wobei die Charakteristika jeweils zumindest entweder einen Amplitudenwert oder eine Frequenzdomänencharakteristik des Signals umfassen.

3. System nach Anspruch 1, wobei der Patientenzustand zumindest entweder einen Anfallszustand, einen Stimmungszustand, einen Bewegungszustand oder einen Patientenhaltungszustand umfasst.

4. System nach Anspruch 1, wobei der Parameter zumindest entweder eine Patientenbewegung oder -aktivität, Herzrate, Atmungsrate, eine elektrodermale Aktivität, thermische Aktivität oder Muskelaktivität umfasst.

5. System nach Anspruch 1, wobei das Mittel, das konfiguriert ist, um die Evaluierungsmetrik zu bestimmen, ein Mittel umfasst, das konfiguriert ist, um auf eine Datenstruktur zuzugreifen, die eine Mehrzahl von Evaluierungsmetriken mit betreffenden Abständen zu der Klassifizierungsgrenze verbindet.

6. System nach Anspruch 1, weiter umfassend ein Mittel (40), das konfiguriert ist, um eine Therapieabgabe an den Patienten basierend auf der Evaluierungsmetrik zu steuern.

7. System nach Anspruch 6, wobei das Mittel, das konfiguriert ist, um die Therapieabgabe an den Patienten zu steuern, zumindest einen Therapieparameterwert basierend auf der Evaluierungsmetrik auswählt, wobei das System weiter ein Therapiemodul umfasst, das eine Therapie an den Patienten basierend auf dem ausgewählten zumindest einen Therapieparameterwert abgibt.

8. System nach Anspruch 6, wobei die Evaluationsmetrik den Abstand umfasst, und wobei das Mittel, das konfiguriert ist, um die Therapieabgabe an den Patientan basierend auf der Evaluierungsmetrik zu steuern, zumindest die Therapieabgabe an den Patienten entweder deaktiviert, aktiviert oder anpasst, wenn die Evaluierungsmetrik niedriger oder gleich zu einem Schwellwert ist.

9. System nach Anspruch 6, wobei die Evaluierungsmetrik den Abstand umfasst, und wobei das Mittel, das konfiguriert ist, um die Therapieabgabe an den Patienten zu steuern, zumindest die Therapieabgabe an den Patienten entweder deaktiviert, aktiviert oder anpasst, wenn die Evaluationsmetrik größer oder gleich zu einem Schwellwert ist.

10. System nach Anspruch 1, weiter umfassend einen Speicher (42), der die Evaluierungsmetrik speichert.

11. System nach Anspruch 1, wobei zumindest entweder das Mittel, das konfiguriert ist, um den Merkmalsvektor basierend auf dem Signal zu bestimmen, das Mittel, das konfiguriert ist, um den auf der Stützvektormaschine basierenden Algorithmus anzuwenden, um den Patientenzustand basierend auf dem Merkmalsvektor zu klassifizieren, das Mittel, das konfiguriert ist, um den Abstand zwischen dem Merkmalsvektor und der Klassifizierungsgrenze zu bestimmen, oder das Mittel, das konfiguriert ist, um eine Evaluierungsmetrik für den Patientenzustand basierend auf dem Abstand zu bestimmen, einen Prozessor umfasst.

12. System nach Anspruch 1, weiter umfassend eine implantierbare medizinische Vorrichtung (16), die das Mittel umfasst, das konfiguriert ist, um das auf den Parameter des Patenten hinweisende Signal zu erzeugen.

**Revendications**

1. Système comportant :

    des moyens configurés pour générer un signal indicatif d'un paramètre d'un patient ;
    des moyens (40) configurés pour déterminer un vecteur d'attributs sur la base du signal ; et
    des moyens (40) configurés pour appliquer un algorithme basé sur une machine à vecteurs de support pour classer un état de patient sur la base du vecteur d'attributs, dans lequel l'algorithme basé sur une machine à vecteurs de support définit une classification ;
    **caractérisé par**
    des moyens (40) configurés pour déterminer une distance entre le vecteur d'attributs et la frontière de classification après classification de l'état du patient sur la base du vecteur d'attributs ; et
    des moyens (40) configurés pour déterminer une grandeur de mesure d'évaluation pour l'état du patient sur la base de la distance, et dans lequel la grandeur de mesure d'évaluation indique une gravité de l'état du patient.

2. Système selon la revendication 1, dans lequel le vecteur d'attributs comporte des valeurs respectives pour au moins deux caractéristiques du signal, dans lequel les caractéristiques comportent chacune au moins un élément parmi une valeur d'amplitude ou une caractéristique de domaine fréquentiel du signal.

3. Système selon la revendication 1, dans lequel l'état du patient comporte au moins un état parmi un état de crise d'épilepsie, un état d'humeur, un état de mouvement ou un état de posture du patient.

4. Système selon la revendication 1, dans lequel le paramètre comporte au moins un élément parmi un mouvement ou une activité du patient, une fréquence cardiaque, une fréquence respiratoire, une activité électrodermique, une activité thermique ou une activité musculaire.

5. Système selon la revendication 1, dans lequel les moyens configurés pour déterminer la grandeur de mesure d'évaluation comportent des moyens configurés pour accéder à une structure de données qui associe une pluralité de grandeurs de mesure d'évaluation à des distances respectives par rapport à la frontière de classification.

6. Système selon la revendication 1, comportant en outre des moyens (40) configurés pour commander l'administration d'une thérapie au patient sur la base de la grandeur de mesure d'évaluation.

7. Système selon la revendication 6, dans lequel les moyens configurés pour commander l'administration d'une thérapie au patient sélectionnent au moins une valeur de paramètre de thérapie sur la base de la grandeur de mesure d'évaluation, le système comportant en outre un module de thérapie qui délivre une thérapie au patient sur la base de la au moins une valeur de paramètre de thérapie sélectionnée.

8. Système selon la revendication 6, dans lequel la grandeur de mesure d'évaluation comporte la distance, et dans lequel les moyens configurés pour commander l'administration d'une thérapie au patient sur la base de la grandeur de mesure d'évaluation exécutent au moins une action parmi une désactivation, une activation ou un ajustement d'administration de thérapie au patient si la grandeur de mesure d'évaluation est inférieure ou égale à une valeur de seuil.

9. Système selon la revendication 6, dans lequel la grandeur de mesure d'évaluation comporte la distance, et dans lequel les moyens configurés pour commander l'administration d'une thérapie au patient sur la base de la grandeur de mesure d'évaluation exécutent au moins une action parmi une désactivation, une activation ou un ajustement d'administration de thérapie au patient si la grandeur de mesure d'évaluation est supérieure ou égale à une valeur de seuil.

10. Système selon la revendication 1, comportant en outre une mémoire (42) qui mémorise la grandeur de mesure d'évaluation.

11. Système selon la revendication 1, dans lequel au moins un moyen parmi les moyens configurés pour déterminer le vecteur d'attributs sur la base du signal, les moyens configurés pour appliquer l'algorithme basé sur une machine à vecteurs de support pour classer l'état du patient sur la base du vecteur d'attributs, les moyens configurés pour déterminer la distance entre le vecteur d'attributs et la frontière de classification, et les moyens configurés pour déterminer une grandeur de mesure d'évaluation pour l'état du patient sur la base de la distance, comporte un processeur.

12. Système selon la revendication 1, comportant en outre un dispositif médical implantable (16) qui comporte les moyens configurés pour générer le signal indicatif du paramètre du patient.

EP 2 429 643 B1

**FIG. 1**

FIG. 2

28

USER INTERFACE
66

USER INPUT
MECHANISM
76

DISPLAY
78

TELEMETRY
MODULE
64

PROCESSOR
60

POWER
SOURCE
68

MEMORY
62

FIG. 3

RECEIVE INDICATIONS OF
FIRST PATIENT STATE AND
SECOND PATIENT STATES — 100

RECEIVE PATIENT
PARAMETER SIGNAL — 102

IDENTIFY SIGNAL PORTIONS
INDICATIVE OF FIRST STATE
AND SECOND STATE — 104

DETERMINE FEATURE
VECTORS FOR IDENTIFIED
SIGNAL PORTIONS — 106

MORE
INDICATIONS OF THE
FIRST AND SECOND
PATIENT
STATES? — 108

YES

NO

USE SVM ALGORITHM TO
DEFINE BOUNDARY
DELINEATING FIRST AND
SECOND PATIENT STATES — 110

**FIG. 4**

**FIG. 5**

FIG. 6

FIG. 7

FIG. 8A

FIG. 8B

FIG. 9

FIG. 10

160 ── RECEIVE PATIENT
PARAMETER SIGNAL

162 ── DETERMINE FEATURE VALUES
FOR FEATURE VECTOR BASED
ON SIGNAL

177 ── FEATURE
VECTORS
APPROACHING
CLASSIFICATION
BOUNDARY?

NO

YES

GENERATE
PROSPECTIVE
PATIENT STATE
INDICATION ── 178

FIG.11

```
┌─────────────────────────────┐
│  RECEIVE PHYSIOLOGICAL      │  160
│  SIGNAL                     │
└─────────────────────────────┘
              │
              ▼
┌─────────────────────────────┐
│  DETERMINE FEATURE VALUES   │  162
│  FOR FEATURE VECTOR BASED   │
│  ON SIGNAL                  │
└─────────────────────────────┘
              │
              ▼
┌─────────────────────────────┐
│  COMPARE FEATURE VECTOR     │  164
│  TO FIRST CLASSIFICATION    │
│  BOUNDARY                   │
└─────────────────────────────┘
              │
              ▼
        FEATURE                        168
   VECTOR CLASSIFIED   ── YES ──▶  ┌──────────────────────┐
   AS FIRST                        │ GENERATE FIRST STATE │
   STATE?         166              │ INDICATION           │
                                   └──────────────────────┘
              │ NO
              ▼
┌─────────────────────────────┐
│  COMPARE FEATURE VECTOR     │  180
│  TO SECOND CLASSIFICATION   │
│  BOUNDARY                   │
└─────────────────────────────┘
              │
              ▼
        FEATURE                        186
   VECTOR CLASSIFIED   ── YES ──▶  ┌──────────────────────┐
   AS SECOND                       │ GENERATE SECOND      │
   STATE?         182              │ STATE INDICATION     │
                                   └──────────────────────┘
              │ NO
              ▼
┌─────────────────────────────┐
│  GENERATE THIRD STATE       │  184
│  INDICATION                 │
└─────────────────────────────┘
```

**FIG. 12**

190 — DETERMINE DISTANCE BETWEEN FEATURE VECTOR AND BOUNDARY

192 — COMPARE DETERMINED DISTANCE TO STORED RANGE OF VALUES

194 — DETERMINE SEVERITY OF PATIENT STATE BASED ON COMPARISON

FIG. 13

**FIG. 14A**

**FIG. 14B**

| DISTANCE | SEVERITY METRIC |
|----------|-----------------|
| D < D1 | 1 |
| D1 ≤ D < D2 | 2 |
| D2 ≤ D < D3 | 3 |
| D ≥ D3 | 4 |

**FIG. 15**

**FIG. 16**

## FIG. 17

| Alg Type | Latency (sec) | Sensitivity | False Det / Day |
|---|---|---|---|
| RBF_2C_2B | 7.3 | 60/61 | 4 |
| Linear_2C_2B | 8.6 | 60/61 | 8 |
| RBF_2C_1B | 8.6 | 53/61 | 9 |
| Linear_2C_1B | 7 | 51/61 | 7 |
| RBF_1C_2B | 8.7 | 60/61 | 8 |
| Linear_1C_2B | 8.33 | 60/61 | 10 |
| BR3 | 7.8 | 57/61 | 36 |
| BR10 | 17 | 43/61 | 5 |

## FIG. 18

| Algorithm | Current Draw |
|---|---|
| ALGORITHM 1 | 12 uA |
| ALGORITHM 2 | 4 uA |
| ALGORITHM 3 | 48 uA |

## FIG. 19

**REFERENCES CITED IN THE DESCRIPTION**

**Patent documents cited in the description**

- WO 2006017153 A
- WO 2004100781 A
- WO 02096282 A
- WO 2005117693 A
- WO 2007054841 A
- US 42606509 A, Giftakis
- US 23621108 A, Kovach
- US 236211 A, Kovach
- US 7385443 B, Denison
- US 97537207 P, Denison
- US 61025503 A, Denison
- US 61083381 A
- US 20090082691 A, Denison
- US 7610083 B, Drew
- US 5995868 A, Dorfmeister
- US 35905509 A, Giftakis
- US 12359037 B, Giftakis
- US 7280867 B, Frei
- US 12236211 B, Kovach